# EUROPEAN PATENT SPECIFICATION

(11) **EP 1 921 161 B1**
(45) Date of publication and mention of the grant of the patent: **13.05.2015**
(21) Application number: 07021823.5
(22) Date of filing: 09.11.2007
(51) Int. Cl.: C12Q 1/68

(54) **Probe, probe set, probe-immobilized carrier, and genetic testing method**
Sonde, Sondensatz, immobilisierter Sondenträger und Genuntersuchungsverfahren
Sonde, ensemble sonde, porteur à sonde immobilisée, et procédé de dépistage génétique

(30) Priority: 10.11.2006 JP 2006306003
(43) Date of publication of application: 14.05.2008
(73) Proprietor: Canon Kabushiki Kaisha, Ohta-ku Tokyo (JP)
(72) Inventor: Kuribayashi, Hideto, Tokyo (JP); Fukui, Toshifumi, Tokyo (JP); Yoshii, Hiroto, Tokyo (JP)
(74) Representative: WESER & Kollegen

(56) References cited:
- WO-A-03/106676
- JP-A- 2006 129 810
- KOSTIC ET AL: "A microbial diagnostic microarray technique for the sensitive detection and identification of pathogenic bacteria in a background of nonpathogens" ANALYTICAL BIOCHEMISTRY, ACADEMIC PRESS, SAN DIEGO, CA, US, vol. 360, no. 2, 30 October 2006 (2006-10-30), pages 244-254, XP005762541 ISSN: 0003-2697
- DORSCH M ET AL: "Rapid identification of Aeromonas species using 16S rDNA targeted oligonucleotide primers: a molecular approach based on screening of environmental isolates" JOURNAL OF APPLIED BACTERIOLOGY, BLACKWELL PUBLISHING LTD., OXFORD, GB, vol. 77, no. 6, December 1994 (1994-12), pages 722-726, XP009097407 ISSN: 0021-8847

### SEQUENCE LISTING

<110> Canon Kabushiki Kaisha
<120> Probe, Probe set, Probe-immobilized carrier and Genetic testing method
<130> 10048405EP01
<150> JP2006-306003
   <151> 2006-11-10
<160> 95
<170> PatentIn version 3.1
<210> 1
   <211> 23
   <212> DNA
   <213> Artificial
<220>
   <223> Primer
<400> 1
   gcggcgtgcc taatacatgc aag 23
<210> 2
   <211> 23
   <212> DNA
   <213> artificial
<220>
   <223> Primer
<400> 2
   gcggcaggcc taacacatgc aag 23
<210> 3
   <211> 23
   <212> DNA
   <213> artificial
<220>
   <223> Primer
<400> 3
   gcggcaggct taacacatgc aag 23
<210> 4
   <211> 23
   <212> DNA
   <213> artificial
<220>
   <223> Primer
<400> 4
   gcggtaggcc taacacatgc aag 23
<210> 5
   <211> 23
   <212> DNA
   <213> artificial
<220>
   <223> Primer
<400> 5
   gcggcgtgct taacacatgc aag 23
<210> 6
   <211> 23
   <212> DNA
   <213> artificial
<220>
   <223> Primer
<400> 6
   gcgggatgcc ttacacatgc aag 23
<210> 7
   <211> 23
   <212> DNA
   <213> artificial
<220>
   <223> Primer
<400> 7
   gcggcatgcc ttacacatgc aag 23
<210> 8
   <211> 23
   <212> DNA
   <213> artificial
<220>
   <223> Primer
<400> 8
   gcggcatgct taacacatgc aag 23
<210> 9
   <211> 23
   <212> DNA
   <213> artificial
<220>
   <223> Primer
<400> 9
   gcggcgtgct taatacatgc aag 23
<210> 10
   <211> 23
   <212> DNA
   <213> artificial
<220>
   <223> Primer
<400> 10
   gcggcaggcc taatacatgc aag 23
<210> 11
   <211> 23
   <212> DNA
   <213> artificial
<220>
   <223> Primer
<400> 11
   gcgggatgct ttacacatgc aag 23
<210> 12
   <211> 23
   <212> DNA
   <213> artificial
<220>
   <223> Primer
<400> 12
   gcggcgtgcc taacacatgc aag 23
<210> 13
   <211> 23
   <212> DNA
   <213> artificial
<220>
   <223> Primer
<400> 13
   gcggcgtgca taacacatgc aag 23
<210> 14
   <211> 23
   <212> DNA
   <213> artificial
<220>
   <223> Primer
<400> 14
   gcggcatgcc taacacatgc aag 23
<210> 15
   <211> 23
   <212> DNA
   <213> artificial
<220>
   <223> Primer
<400> 15
   gcggcgcgcc taacacatgc aag 23
<210> 16
   <211> 23
   <212> DNA
   <213> artificial
<220>
   <223> Primer
<400> 16
   gcggcgcgct taacacatgc aag 23
<210> 17
   <211> 23
   <212> DNA
   <213> artificial
<220>
   <223> Primer
<400> 17
   gcgtcatgcc taacacatgc aag 23
<210> 18
   <211> 23
   <212> DNA
   <213> artificial
<220>
   <223> Primer
<400> 18
   gcgataggct taacacatgc aag 23
<210> 19
   <211> 23
   <212> DNA
   <213> artificial
<220>
   <223> Primer
<400> 19
   gcgacaggct taacacatgc aag 23
<210> 20
   <211> 23
   <212> DNA
   <213> artificial
<220>
   <223> Primer
<400> 20
   gctacaggct taacacatgc aag 23
<210> 21
   <211> 23
   <212> DNA
   <213> artificial
<220>
   <223> Primer
<400> 21
   acagaatgct taacacatgc aag 23
<210> 22
   <211> 22
   <212> DNA
   <213> artificial
<220>
   <223> Primer
<400> 22
   atccagccgc accttccgat ac 22
<210> 23
   <211> 22
   <212> DNA
   <213> artificial
<220>
   <223> Primer
<400> 23
   atccaaccgc aggttcccct ac 22
<210> 24
   <211> 22
   <212> DNA
   <213> artificial
<220>
   <223> Primer
<400> 24
   atccagccgc aggttcccct ac 22
<210> 25
   <211> 22
   <212> DNA
   <213> artificial
<220>
   <223> Primer
<400> 25
   atccagccgc accttccggt ac 22
<210> 26
   <211> 22
   <212> DNA
   <213> artificial
<220>
   <223> Primer
<400> 26
   atccagcgcc aggttcccct ag 22
<210> 27
   <211> 22
   <212> DNA
   <213> artificial
<220>
   <223> Primer
<400> 27
   atccagccgc aggttctcct ac 22
<210> 28
   <211> 22
   <212> DNA
   <213> artificial
<220>
   <223> Primer
<400> 28
   atccagccgc acgttcccgt ac 22
<210> 29
   <211> 23
   <212> DNA
   <213> artificial
<220>
   <223> Primer
<400> 29
   taccttgtta cgacttcacc cca 23
<210> 30
   <211> 23
   <212> DNA
   <213> artificial
<220>
   <223> Primer
<400> 30
   taccttgtta cgacttcgtc cca 23
<210> 31
   <211> 23
   <212> DNA
   <213> artificial
<220>
   <223> Primer
<400> 31
   taccttgtta cgacttagtc cca 23
<210> 32
   <211> 23
   <212> DNA
   <213> artificial
<220>
   <223> Primer
<400> 32
   taccttgtta cgacttagcc cca 23
<210> 33
   <211> 23
   <212> DNA
   <213> artificial
<220>
   <223> Primer
<400> 33
   taccttgtta cgacttagtc cta 23
<210> 34
   <211> 23
   <212> DNA
   <213> artificial
<220>
   <223> Primer
<400> 34
   taccttgtta cgacttagcc cta 23
<210> 35
   <211> 28
   <212> DNA
   <213> Artificial
<220>
   <223> probe
<400> 35
   tcatcttgag gtatggaagg gaaagtgg 28
<210> 36
   <211> 28
   <212> DNA
   <213> artificial
<220>
   <223> probe
<400> 36
   gtgttaggtg tctggaataa tctgggtg 28
<210> 37
   <211> 25
   <212> DNA
   <213> artificial
<220>
   <223> probe
<400> 37
   accaagtctt gacatattac ggcgg 25
<210> 38
   <211> 26
   <212> DNA
   <213> artificial
<220>
   <223> probe
<400> 38
   aaggattccg gtaaaggatg gggatg 26
<210> 39
   <211> 26
   <212> DNA
   <213> artificial
<220>
   <223> probe
<400> 39
   tggaaacatg tcagtgagca atcacc 26
<210> 40
   <211> 27
   <212> DNA
   <213> artificial
<220>
   <223> probe
<400> 40
   aagaatttcg gttatcgatg gggatgc 27
<210> 41
   <211> 29
   <212> DNA
   <213> artificial
<220>
   <223> probe
<400> 41
   aagttttcca cgtgtggaat tttgtatgt 29
<210> 42
   <211> 24
   <212> DNA
   <213> artificial
<220>
   <223> probe
<400> 42
   aaggcagcta cctggtgaca ggat 24
<210> 43
   <211> 30
   <212> DNA
   <213> artificial
<220>
   <223> probe
<400> 43
   aatatcaaag gtgagccagt acaggatgga 30
<210> 44
   <211> 28
   <212> DNA
   <213> artificial
<220>
   <223> probe
<400> 44
   ccgtagtaag ctcttgaaac tgggagac 28
<210> 45
   <211> 28
   <212> DNA
   <213> artificial
<220>
   <223> probe
<400> 45
   tcccaatgac atctccttaa tcggagag 28
<210> 46
   <211> 27
   <212> DNA
   <213> artificial
<220>
   <223> probe
<400> 46
   aaccaaagga gcaatccgct atgagat 27
<210> 47
   <211> 25
   <212> DNA
   <213> artificial
<220>
   <223> probe
<400> 47
   gagcgtaggc ggatgattaa gtggg 25
<210> 48
   <211> 29
   <212> DNA
   <213> artificial
<220>
   <223> probe
<400> 48
   cccttgcatt actcttaatc gaggaaatc 29
<210> 49
   <211> 22
   <212> DNA
   <213> artificial
<220>
   <223> probe
<400> 49
   ggaaagccca gacggcaagg ga 22
<210> 50
   <211> 26
   <212> DNA
   <213> artificial
<220>
   <223> probe
<400> 50
   cctctcaagc gggatctcta atccga 26
<210> 51
   <211> 23
   <212> DNA
   <213> artificial
<220>
   <223> probe
<400> 51
   tgccccatgt tgccagcatt agg 23
<210> 52
   <211> 28
   <212> DNA
   <213> artificial
<220>
   <223> probe
<400> 52
   ttttcgcatg gaggaatcat gaaagcta 28
<210> 53
   <211> 21
   <212> DNA
   <213> artificial
<220>
   <223> probe
<400> 53
   agatgcgccg gtgccctttc g 21
<210> 54
   <211> 26
   <212> DNA
   <213> artificial
<220>
   <223> probe
<400> 54
   agtcgggaag aagtcagtga cggtac 26
<210> 55
   <211> 24
   <212> DNA
   <213> artificial
<220>
   <223> probe
<400> 55
   gagtacgtgc gcaagcatga aact 24
<210> 56
   <211> 24
   <212> DNA
   <213> artificial
<220>
   <223> probe
<400> 56
   gaagactgcc cgcaagggtt gtaa 24
<210> 57
   <211> 25
   <212> DNA
   <213> artificial
<220>
   <223> probe
<400> 57
   gtgtactgga ggtacgtgga acgtg 25
<210> 58
   <211> 24
   <212> DNA
   <213> artificial
<220>
   <223> probe
<400> 58
   gcatgaggct gagaggtctc ttcc 24
<210> 59
   <211> 28
   <212> DNA
   <213> artificial
<220>
   <223> probe
<400> 59
   ttatagctgt aagataggca tgcgtccc 28
<210> 60
   <211> 26
   <212> DNA
   <213> artificial
<220>
   <223> probe
<400> 60
   aacgggcgat acgagtattg cattga 26
<210> 61
   <211> 25
   <212> DNA
   <213> artificial
<220>
   <223> probe
<400> 61
   atataccgtc aagcttccac agcga 25
<210> 62
   <211> 25
   <212> DNA
   <213> artificial
<220>
   <223> probe
<400> 62
   tttgaaaggc gcttttgcgt cactg 25
<210> 63
   <211> 30
   <212> DNA
   <213> artificial
<220>
   <223> probe
<400> 63
   caaggatgag agtagaacgt tcatcccttg 30
<210> 64
   <211> 30
   <212> DNA
   <213> artificial
<220>
   <223> probe
<400> 64
   caaggatgag agtagaatgt tcatcccttg 30
<210> 65
   <211> 27
   <212> DNA
   <213> artificial
<220>
   <223> probe
<400> 65
   cctgggaatg gcatctaaga ctggtca 27
<210> 66
   <211> 28
   <212> DNA
   <213> artificial
<220>
   <223> probe
<400> 66
   gaggaaggcg ttgatgctaa tatcatca 28
<210> 67
   <211> 24
   <212> DNA
   <213> artificial
<220>
   <223> probe
<400> 67
   gagcaaagca ggggaacttc ggtc 24
<210> 68
   <211> 24
   <212> DNA
   <213> artificial
<220>
   <223> probe
<400> 68
   gttggggcct ttgaggcttt agtg 24
<210> 69
   <211> 26
   <212> DNA
   <213> artificial
<220>
   <223> probe
<400> 69
   tgggagagga tggtagaatt ccaggt 26
<210> 70
   <211> 26
   <212> DNA
   <213> artificial
<220>
   <223> probe
<400> 70
   tcgatgacgg catcagattc gaagca 26
<210> 71
   <211> 24
   <212> DNA
   <213> artificial
<220>
   <223> probe
<400> 71
   aatgtaggcg cccaacgtct gact 24
<210> 72
   <211> 24
   <212> DNA
   <213> artificial
<220>
   <223> probe
<400> 72
   atgttgaggt ccttcgggac tcct 24
<210> 73
   <211> 31
   <212> DNA
   <213> artificial
<220>
   <223> probe
<400> 73
   ggaagtaact agaatatgta gtgtagcggt g 31
<210> 74
   <211> 24
   <212> DNA
   <213> artificial
<220>
   <223> probe
<400> 74
   gccagtgcaa actgtgagga aggt 24
<210> 75
   <211> 25
   <212> DNA
   <213> artificial
<220>
   <223> probe
<400> 75
   gggtaggggt cctgagaggg agatc 25
<210> 76
   <211> 24
   <212> DNA
   <213> artificial
<220>
   <223> probe
<400> 76
   gagtgccttc gggaatcaga acac 24
<210> 77
   <211> 24
   <212> DNA
   <213> artificial
<220>
   <223> probe
<400> 77
   ctgcaagcta gcgatagtga gcga 24
<210> 78
   <211> 25
   <212> DNA
   <213> artificial
<220>
   <223> probe
<400> 78
   cgatggatag gggttctgag aggaa 25
<210> 79
   <211> 25
   <212> DNA
   <213> artificial
<220>
   <223> probe
<400> 79
   tgcggctcaa ccgtaaaatt gcagt 25
<210> 80
   <211> 25
   <212> DNA
   <213> artificial
<220>
   <223> probe
<400> 80
   tgtggctcaa ccatagaatt gccgt 25
<210> 81
   <211> 26
   <212> DNA
   <213> artificial
<220>
   <223> probe
<400> 81
   gtacctgtaa gaaagacggc cttcgt 26
<210> 82
   <211> 27
   <212> DNA
   <213> artificial
<220>
   <223> probe
<400> 82
   ctgccgagtg atgtaatgtc acttttc 27
<210> 83
   <211> 22
   <212> DNA
   <213> artificial
<220>
   <223> probe
<400> 83
   tcggaggttt caagaccgtc gg 22
<210> 84
   <211> 27
   <212> DNA
   <213> artificial
<220>
   <223> probe
<400> 84
   accaaaggag caatccgcta tgagatg 27
<210> 85
   <211> 26
   <212> DNA
   <213> artificial
<220>
   <223> probe
<400> 85
   atcaaaggtg agccagtaca ggatgg 26
<210> 86
   <211> 31
   <212> DNA
   <213> artificial
<220>
   <223> probe
<400> 86
   attaaaggag taatccgcta tgagatggac c 31
<210> 87
   <211> 26
   <212> DNA
   <213> artificial
<220>
   <223> probe
<400> 87
   gggctaatac cggataggag ctcctg 26
<210> 88
   <211> 26
   <212> DNA
   <213> artificial
<220>
   <223> probe
<400> 88
   aagcgtgagt gacggtaatg ggtaaa 26
<210> 89
   <211> 25
   <212> DNA
   <213> artificial
<220>
   <223> probe
<400> 89
   atcgcgtcgg aagtgtaatc ttggg 25
<210> 90
   <211> 29
   <212> DNA
   <213> artificial
<220>
   <223> probe
<400> 90
   tggagcaaat ctataaaata tgtcccagt 29
<210> 91
   <211> 22
   <212> DNA
   <213> artificial
<220>
   <223> probe
<400> 91
   acagtggaat cagcgactgg gg 22
<210> 92
   <211> 28
   <212> DNA
   <213> artificial
<220>
   <223> probe
<400> 92
   gcctaatacg tatcaactgt gacgttac 28
<210> 93
   <211> 28
   <212> DNA
   <213> artificial
<220>
   <223> probe
<400> 93
   gcctaatacg tgtcaactgt gacgttac 28
<210> 94
   <211> 27
   <212> DNA
   <213> artificial
<220>
   <223> probe
<400> 94
   gctttcgata cgggttgact tgaggaa 27
<210> 95
   <211> 1507
   <212> DNA
   <213> Aeromonas hydrophila
<400> 95
   ctcagattga acgctggcgg caggcctaac acatgcaagt cgagcggcag cgggaaagta      60
   gcttgctact tttgccggcg agcggcggac gggtgagtaa tgcctgggaa attgcccagt     120
   cgagggggat aacagttgga aacgactgct aataccgcat acgccctacg ggggaaagca     180
   ggggaccttc gggccttgcg cgattggata tgcccaggtg ggattagcta gttggtgagg     240
   taatggctca ccaaggcgac gatccctagc tggtctgaga ggatgatcag ccacactgga     300
   actgagacac ggtccagact cctacgggag gcagcagtgg ggaatattgc acaatggggg     360
   aaaccctgat gcagccatgc cgcgtgtgtg aagaaggcct tcgggttgta aagcactttc     420
   agcgaggagg aaaggttgat gcctaatacg tatcaactgt gacgttactc gcagaagaag     480
   caccggctaa ctccgtgcca gcagccgcgg taatacggag ggtgcaagcg ttaatcggaa     540
   ttactgggcg taaagcgcac gcaggcggtt ggataagtta gatgtgaaag ccccgggctc     600
   aacctgggaa ttgcatttaa aactgtccag ctagagtctt gtagaggggg gtagaattcc     660
   aggtgtagcg gtgaaatgcg tagagatctg gaggaatacc ggtggcgaag gcggccccct     720
   ggacaaagac tgacgctcag gtgcgaaagc gtggggagca aacaggatta gataccctgg     780
   tagtccacgc cgtaaacgat gtcgatttgg aggctgtgtc cttgagacgt ggcttccgga     840
   gctaacgcgt taaatcgacc gcctggggag tacggccgca aggttaaaac tcaaatgaat     900
   tgacgggggc ccgcacaagc ggtggagcat gtggtttaat tcgatgcaac gcgaagaacc     960
   ttacctggcc ttgacatgtc tggaatcctg cagagatgcg ggagtgcctt cgggaatcag    1020
   aacacaggtg ctgcatggct gtcgtcagct cgtgtcgtga gatgttgggt taagtcccgc    1080
   aacgagcgca acccctgtcc tttgttgcca gcacgtaatg gtgggaactc aagggagact    1140
   gccggtgata aaccggagga aggtggggat gacgtcaagt catcatggcc cttacggcca    1200
   gggctacaca cgtgctacaa tggcgcgtac agagggctgc aagctagcga tagtgagcga    1260
   atcccaaaaa gcgcgtcgta gtccggatcg gagtctgcaa ctcgactccg tgaagtcgga    1320
   atcgctagta atcgcaaatc agaatgttgc ggtgaatacg ttcccgggcc ttgtacacac    1380
   cgcccgtcac accatgggag tgggttgcac cagaagtaga tagcttaacc ttcgggaggg    1440
   cgtttaccac ggtgtgattc atgactgggg tgaagtcgta acaaggtaac cctaggggaa    1500
   cctgcgg                                                              1507

## Description

### BACKGROUND OF THE INVENTION

### Field of the Invention

The present invention relates to a probe and a probe set for detecting a gene of infectious disease pathogenic bacterium, *Aeromonas hydrophila,* which are useful for detection and identification of the causative organism of an infectious disease, a probe-immobilized carrier on which the probe or the probe set is immobilized, a genetic testing method using the probe-immobilized carrier, and a genetic testing kit to be used for the method.

### Description of the Related Art

Heretofore, reagents for and methods of quickly and accurately detecting the causative organisms of infectious diseases in analytes have been proposed. For instance, Japanese Patent Application Laid-Open No. H08-089254 discloses oligonucleotides having specific base sequences, which can be respectively used as probes and primers for detecting pathogenic bacteria of candidiasis and aspergillosis, and a method of detecting target bacteria using such oligonucleotides. In addition, the same patent document also discloses a set of primers used for concurrently amplifying a plurality of target bacteria by PCR. In other words, those primers are used for the PCR amplification of nucleic acid fragments from fungi, which serve as a plurality of targets, in an analyte. Target fungal species in the analyte can be identified by detecting the presence of a specific part of the sequence by a hybridization assay using probes specific to the respective fungi and the nucleic acid fragments amplified by the respective primers.

On the other hand, the method to use probe array in which probes having sequences complementary to the respective base sequences are arranged at intervals on a solid support is known as a method capable of simultaneously detecting a plurality of oligonucleotides having different base sequences (Japanese Patent Application Laid-Open No. 2004-313181).

### SUMMARY OF THE INVENTION

However, it is no easy task to design a probe for specifically detecting a gene of an infectious disease pathogenic bacterium in a sample. That is, as well as the target gene, the sample may further contain genes of other infectious disease pathogenic bacteria. Thus, it is no easy task to design the probe that specifically detects the gene of the infectious disease pathogenic bacterium while suppressing the cross contamination which is the influence of the presence of the genes of other infectious disease pathogenic bacteria. Under such circumstances, the inventors of the present invention have studied for obtaining a probe which allows accurate detection of a gene of an infectious disease pathogenic bacterium as mentioned hereinbelow while maintaining the cross contamination level low even when a sample in which genes of different bacteria are present is used. As a result, the inventors of the present invention have finally found a plurality of probes capable of precisely detecting the gene of the infectious disease pathogenic bacterium, *Aeromonas hydrophila.*

A first object of the present invention is to provide a probe and a probe set, which can precisely identify a gene of a target bacterium from an analyte in which various bacteria are concurrently present. Another object of the present invention is to provide a probe-immobilized carrier which can be used for precisely identifying a target bacterium from an analyte in which various bacteria are concurrently present. Still another object of the present invention is to provide a genetic testing method for detecting a target bacterium, which can quickly and precisely detect the target bacterium from various bacteria in an analyte when they are present therein, and a kit for such a method.

These objects are achieved by the probe-immobilized carriers according to claims 1 and 6, the detection kits according to claims 3 and 5, the detection method according to claim 7, and the probe set according to claim 9. The other claims relate to further developments.

WO03106676 discloses probes on a DNA chip for the detection of Aeromonas hydrophila, which targets the same 16S sRNA region as the present invention and allows the detection and distinction from numerous microbial pathogens. The probes disclosed in WO03106676 are all 50 nucleotides in length.

According to the present invention, when an analyte is infected with the above-mentioned causative bacterium, the bacterium can be more quickly and precisely identified from the analyte even if the analyte is simultaneously and complexly infected with other bacteria in addition to the above-mentioned bacterium. In particular, *Aeromonas hydrophila* can be detected while precisely distinguishing it from *Escherichia coli* which may otherwise cause cross contamination.

Further features of the present invention will become apparent from the following description of exemplary embodiments with reference to the attached drawings.

### BRIEF DESCRIPTION OF THE DRAWINGS

FIG. 1 is a diagram illustrating a 1st PCR protocol.
FIG. 2 is a diagram illustrating a 2nd PCR protocol.

### DESCRIPTION OF THE EMBODIMENTS

The inventors of the present invention have obtained almost all of bacteria (represented by (1) to (80) below), which have been known as septicemia pathogenic bacteria so far, from the respective depository institutions and identified the 16S rRNA gene sequences of all the bacteria.

Subsequently, while making a comparison of all the identified sequences, probe sequences for *Aeromonas hydrophila* were investigated in detail and the probes of the present invention, which can identify *Aeromonas hydrophila,* have finally been found out.

| | | |
|---|---|---|
| (1) | *Staphylococcus aureus* | (ATCC12600) |
| (2) | *Staphylococcus epidermidis* | (ATCC14990) |
| (3) | *Escherichia coli* | (ATCC11775) |
| (4) | *Klebsiella pneumoniae* | (ATCC13883) |
| (5) | *Pseudomonas aeruginosa* | (ATCC10145) |
| (6) | *Serratia marcescens* | (ATCC13380) |
| (7) | *Streptococcus pneumoniae* | (ATCC33400) |
| (8) | *Haemophilus influenzae* | (ATCC33391) |
| (9) | *Enterobacter cloacae* | (ATCC13047) |
| (10) | *Enterococcus faecalis* | (ATCC19433) |
| (11) | *Staphylococcus haemolyticus* | (ATCC29970) |
| (12) | *Staphylococcus hominis* | (ATCC27844) |
| (13) | *Staphylococcus saprophyticus* | (ATCC15305) |
| (14) | *Streptococcus agalactiae* | (ATCC13813) |
| (15) | *Streptococcus mutans* | (ATCC25175) |
| (16) | *Streptococcus pyogenes* | (ATCC12344) |
| (17) | *Streptococcus sanguinis* | (ATCC10556) |
| (18) | *Enterococcus avium* | (JCM8722) |
| (19) | *Enterococcus faecium* | (ATCC19434) |
| (20) | *Pseudomonas fluorescens* | (ATCC13525) |
| (21) | *Pseudomonas putida* | (ATCC12633) |
| (22) | *Burkholderia cepacia* | (JCM5964) |
| (23) | *Stenotrophomonas maltophilia* | (ATCC13637) |
| (24) | *Acinetobacter baumannii* | (ATCC19606) |
| (25) | *Acinetobacter calcoaceticus* | (ATCC23055) |
| (26) | *Achromobacter xylosoxidans* | (ATCC27061) |
| (27) | *Vibrio vulnificus* | (ATCC27562) |
| (28) | *Salmonella choleraesuis* | (JCM1651) |
| (29) | *Citrobacter freundii* | (ATCC8090) |
| (30) | *Klebsiella oxytoca* | (ATCC13182) |
| (31) | *Enterobacter aerogenes* | (ATCC13048) |
| (32) | *Hafnia alvei* | (ATCC13337) |
| (33) | *Serratia liquefaciens* | (ATCC27592) |
| (34) | *Proteus mirabilis* | (ATCC29906) |
| (35) | *Proteus vulgaris* | (ATCC33420) |
| (36) | *Morganella morganii* | (ATCC25830) |
| (37) | *Providencia rettgeri* | (JCM1675) |
| (38) | *Aeromonas hydrophila* | (JCM1027) |
| (39) | *Aeromonas sobria* | (ATCC43979) |
| (40) | *Gardnerella vaginalis* | (ATCC14018) |
| (41) | *Corynebacterium diphtheriae* | (ATCC2701) |
| (42) | *Legionella pneumophila* | (ATCC33152) |
| (43) | *Bacillus cereus* | (ATCC14579) |
| (44) | *Bacillus subtilis* | (ATCC6051) |
| (45) | *Mycobacterium kansasii* | (ATCC12478) |
| (46) | *Mycobacterium intracellulare* | (ATCC13950) |
| (47) | *Mycobacterium chelonae* | (ATCC35752) |
| (48) | *Nocardia asteroids* | (ATCC19247) |
| (49) | *Bacteroides fragilis* | (JCM11019) |
| (50) | *Bacteroides thetaiotaomicron* | (JCM5827) |
| (51) | *Clostridium difficile* | (ATCC51695) |
| (52) | *Clostridium perfringens* | (JCM1290) |
| (53) | *Eggerthella lenta* | (JCM10763) |
| (54) | *Fusobacterium necrophorum* | (JCM3718) |
| (55) | *Fusobacterium nucleatum* | (ATCC25586) |
| (56) | *Lactobacillus acidophilus* | (ATCC4356) |
| (57) | *Anaerococcus prevotii* | (JCM6490) |
| (58) | *Peptoniphilus asaccharolyticus* | (JCM8143) |
| (59) | *Porphyromonas asaccharolytica* | (JCM6326) |
| (60) | *Porphyromonas gingivalis* | (JCM8525) |
| (61) | *Prevotella denticola* | (ATCC38184) |
| (62) | *Propionibacterium acnes* | (JCM6473) |
| (63) | *Acinetobacter johnsonii* | (ATCC17909) |
| (64) | *Acinetobacter junii* | (ATCC17908) |
| (65) | *Aeromonas schubertii* | (ATCC43700) |
| (66) | *Aeromonas veronii* | (ATCC35624) |
| (67) | *Bacteroides distasonis* | (ATCC8503) |
| (68) | *Bacteroides vulgatus* | (ATCC8482) |
| (69) | *Campylobacter coli* | (ATCC33559) |
| (70) | *Campylobacter hyointestinalis* | (ATCC35217) |
| (71) | *Campylobacter jejuni* | (ATCC33560) |
| (72) | *Flavobacterium aquatile* | (ATCC11947) |
| (73) | *Flavobacterium mizutaii* | (ATCC33299) |
| (74) | *Peptococcus niger* | (ATCC27731) |
| (75) | *Propionibacterium avidum* | (ATCC25577) |
| (76) | *Propionibacterium freudenreichii* | (ATCC6207) |
| (77) | *Propionibacterium granulosum* | (ATCC25564) |
| (78) | *Clostridium butyricum* | (ATCC13949) |
| (79) | *Flavobacterium hydatis* | (NBRC14958) |
| (80) | *Flavobacterium johnsoniae* | (NBRC14942) |

The deposition numbers of the bacterial species obtained are shown in the respective parentheses on the right side in the above. Bacterial species having deposition numbers beginning with "ATCC", "JCM" and "NBRC" are available from American Type Culture Collection, Japan Collection of Microorganisms (RIKEN BioResource Center) and National Board for Respiratory Care, respectively.

The present invention provides an oligonucleotide probe for identifying an infectious disease pathogenic bacterium (hereinafter, simply referred to as a probe) and a probe set including a combination of two or more probes. The use of such a probe or a probe set allows the detection of the following bacterium which will cause inflammation by infection.

### [Bacterial Name]

### Aeromonas hydrophila

That is, the probe of the present invention can detect the 16S rRNA gene sequence among genes of the above-mentioned bacterium, having the following sequences:
(A) a probe having a base sequence represented by GCCTAATACGTATCAACTGTGACGTTAC (SEQ ID NO. 92);
(B) a probe having a base sequence represented by GCCTAATACGTGTCAACTGTGACGTTAC (SEQ ID NO. 93);
(C) a probe having a complementary sequence of the base sequence represented by SEQ ID NO. 92;
(D) a probe having a complementary sequence of the base sequence represented by SEQ ID NO. 93;
(E) a probe having a modified sequence obtained by base deletion, substitution, or addition on the base sequence represented by SEQ ID NO. 92 as far as it retains the function of a probe for detecting the gene of *Aeromonas hydrophila;*
(F) a probe having a modified sequence obtained by base deletion, substitution, or addition on the base sequence represented by SEQ ID NO. 93 as far as it retrains the function of a probe for detecting the gene of *Aeromonas hydrophila;*
(G) a probe having a modified sequence obtained by base deletion, substitution, or addition on the complementary sequence of the base sequence represented by SEQ ID NO. 92 as far as it retains the function of a probe for detecting the gene of *Aeromonas hydrophila;* and
(H) a probe having a modified sequence obtained by base deletion, substitution, or addition on the complementary sequence of the base sequence represented by SEQ ID NO. 93 as far as it retains the function of a probe for detecting the gene of *Aeromonas hydrophila.*

The probe set can be formed using at least two of those probes.

The functions of those probes significantly depend on the specificity of each probe sequence corresponding to the target nucleic acid sequence of interest. The specificity of a probe sequence can be evaluated from the degree of coincidence of bases with the target nucleic acid sequence and the probe sequence. Further, when a plurality of probes constitute a probe set, the variance of melting temperatures among the probes may affect the performance of the probe set.

For designing a probe sequence, a region showing a high specificity to a specific bacterial species of interest regardless of any differences in strain is selected. The region contains three or more bases which are not coincident with corresponding bases in the sequences of any other bacterial species. The probe sequence is designed so that the melting temperature between the probe sequence and the corresponding sequence of the specific bacterial species of interest will differ by 10°C or more from the melting temperatures between the probe sequence and the corresponding sequences of any other bacterial species. Further, one or more bases can be deleted or added so that the respective probes immobilized on a single carrier may have melting temperatures within a predetermined range.

The inventors of the present invention found out by experiments that the hybridization intensity of a probe will not be significantly attenuated if 80% or more of the base sequence is consecutively conserved. It can therefore be concluded, from the finding, such that any sequences modified from the probe sequences disclosed in the specification will have a sufficient probe function if 80% or more of the base sequence of the probe is consecutively conserved.

The above-mentioned modified sequences may include any variation as far as it does not impair the probe's function, or any variation as far as it hybridizes with a nucleic acid sequence of interest as a detection target. Above all, it is desirable to include any variation as far as it can hybridize with a nucleic acid sequence of interest as a detection target under stringent conditions. Preferable hybridization conditions confining the variation include those represented in examples as described below. Here, the term "detection target" used herein may be one included in a sample to be used in hybridization, which may be a unique base sequence to the infectious disease pathogenic bacterium, or may be a complementary sequence to the unique sequence. Further, the variation may be a modified sequence obtained by deletion, substitution, or addition of at least one base as far as it retains a function as the probe.

Those probe sequences are only specific to the DNA sequence coding for the 16S rRNA of the above-mentioned bacterium, so sufficient hybridization sensitivity to the sequence will be expected even under stringent conditions. In addition, any of those probe sequences forms a stable hybridized product through a hybridization reaction thereof with a target analyte even when the probe sequences are immobilized on a carrier, which is designed to produce an excellent result.

Further, a probe-immobilized carrier (e.g., DNA chip), on which the probe for detecting the infectious disease pathogenic bacterium of the present invention, can be obtained by supplying the probe on a predetermined position on the carrier and immobilizing the probe thereon. Various methods can be used for supplying the probe to the carrier. Among them, for example, a method, which can be suitably used, is to keep a surface state capable of immobilizing the probe on the carrier through a chemical bonding (e.g., covalent bonding) and a liquid containing the probe is then provided on a predetermined position by an inkjet method. Such a method allows the probe to be hardly detached from the carrier and exerts an additional effect of improving the sensitivity. In other words, when a stamping method conventionally used and called the Stanford method is employed to make a DNA chip, the resultant DNA chip has a disadvantage such that the applied DNA tends to be peeled off. Another one of the methods of forming DNA chips is to carry out the arrangement of probes by the synthesis of DNA on the surface of a carrier (e.g., DNA chip from Affymetrix Co., Ltd.). In such a method of synthesizing probes on a carrier, it is difficult to make equal the amount of synthesized DNA for each probe sequence. Thus, the amount of immobilized probe per immobilization area (spot) for each probe tends to vary considerably. Such variations in amounts of the respective immobilized probes may cause incorrect evaluation on the results of the detection with those probes. Based on this fact, the probe carrier of the present invention is preferably prepared using the above-mentioned inkjet method. The inkjet method as described above has an advantage such that the probe can be stably immobilized on the carrier and hardly detaching from the carrier to efficiently provide a probe carrier which can carry out detection with high sensitivity and high accuracy.

In addition, a probe set may include at least two selected from the group consisting of SEQ ID NOS. 92 to 93 as described above and the complementary sequences thereof and sequences obtained by base deletion, substitution, or addition on those sequences as far as they retain the function of a probe for detecting the gene of *Aeromonas hydrophila.* In this case, the accuracy of detecting the *Aeromonas hydrophila* gene can be further improved.

Hereinafter, preferred embodiments of the present invention will be described in detail.

Test objects to be tested using probe carriers (e.g., DNA chips) in which the probes of the present invention are immobilized on carriers include those originated from humans and animals such as domestic animals. For example, a test object is any of those which may contain bacteria, including: any body fluids such as blood, cerebrospinal fluid, expectorated sputum, gastric juice, vaginal discharge, and oral mucosal fluid; and excretions such as urine and feces. All media, which can be contaminated with bacteria, can be also subjected to a test using a DNA chip. Such media include: food, drink water and water in the natural environment such as hot spring water, which may cause food poisoning by contamination; filters of air cleaners and the like; and so on. Animals and plants, which should be quarantined in import/export, are also used as analytes of interest.

When the sample as described above can be directly used in reaction with the DNA chip, it is used as an analyte to react with the DNA chip and the result of the reaction is then analyzed. Alternatively, when the sample cannot be directly reacted with the DNA chip, the sample was subjected to extraction, purification, and other procedures for obtaining a target substance if required and then provided as an analyte to carry out a reaction with the DNA chip. For instance, when the sample contains a target nucleic acid, an extract, which may be assumed to contain such a target nucleic acid, is prepared from a sample, and then washed, diluted, or the like to obtain an analyte solution followed by reaction with the DNA chip. Further, as a target nucleic acid is included in an analyte obtained by carrying out various amplification procedures such as PCR amplification, the target nucleic acid may be amplified and then reacted with a DNA chip. Such analytes of amplified nucleic acids include the following ones: (a) An amplified analyte prepared by using a PCR-reaction primer designed for detecting rRNA gene. (b) An amplified analyte prepared by an additional PCR reaction or the like from a PCR-amplified product. (c) An analyte prepared by an amplification method other than PCR. (d) An analyte labeled for visualization by any of various labeling methods.

Further, a carrier used for preparing a probe-immobilized carrier, such as a DNA chip, may be any of those that satisfy the property of carrying out a solid phase/liquid phase reaction of interest. Examples of the carrier include: flat substrates such as a glass substrate, a plastic substrate, and a silicon wafer; a three-dimensional structure having an irregular surface; and a spherical body such as a bead, and rod-, cord-, and thread-shaped structures. The surface of the carrier may be processed such that a probe can be immobilized thereon. Especially, a carrier prepared by introducing a functional group to its surface to enable chemical reaction has a preferable form from the viewpoint of reproducibility because the probe is stably bonded in the process of hybridization reaction.

Various methods can be employed for the immobilization of probes. An example of such a method is to use a combination of a maleimide group and a thiol (-SH) group. In this method, a thiol (-SH) group is bonded to the terminal of a probe, and a process executed in advance to make the carrier (solid) surface have a maleimide group. Accordingly, the thiol group of the probe supplied to the carrier surface reacts with the maleimide group on the carrier surface to form a covalent bond, whereby the probe is immobilized.

Introduction of the maleimide group can utilize a process of firstly allowing a reaction between a glass substrate and an aminosilane coupling agent and then introducing a maleimide group onto the glass substrate by a reaction of the amino group with an EMCS reagent (N-(6-maleimidocaproyloxy)succinimide, available from Dojindo). Introduction of the thiol group to a DNA can be carried out using 5'-Thiol-Modifier C6 (available from Glen Research) when the DNA is synthesized by an automatic DNA synthesizer.

Instead of the above-described combination of a thiol group and a maleimide group, a combination of, e.g., an epoxy group (on the solid phase) and an amino group (nucleic acid probe terminal), can also be used as a combination of functional groups to be used for immobilization. Surface treatments using various kinds of silane coupling agents are also effective. A probe in which a functional group which can react with a functional group introduced by a silane coupling agent is introduced is used. A method of applying a resin having a functional group can also be used.

The detection of the gene of the infectious disease pathogenic bacterium by using the probe-immobilized carrier of the present invention can be carried out by a genetic testing method including the steps of:
(i) reacting an analyte with a probe-immobilized carrier on which the probe of the present invention is immobilized;
(ii) detecting the presence or absence of the reaction of a nucleic acid in the analyte with the probe on the probe-immobilized carrier, or detecting the strength of the hybridization reaction of a nucleic acid in the analyte with the probe on the probe-immobilized carrier; and
(iii) specifying the probe having reacted with the nucleic acid in the analyte when the reaction of the probe with the nucleic acid in the analyte is detected and specifying the gene of the infectious disease pathogenic bacterium in the analyte based on the nucleic acid sequence of the probe.

The probe to be immobilized on the probe-immobilized carrier is at least one of the above-mentioned items (A) to (H). On the carrier, probes for detecting bacterial species other than *Aeromonas hydrophila* may be immobilized as other probes, depending on the purpose of test. In this case, the other probes may be those capable of detecting the bacterial species other than *Aeromonas hydrophila* without causing cross contamination and the use of such probes allows simultaneous detection of a plurality of bacterial species with high accuracy.

Further, as described above, when the 16S rRNA gene sequence of an infectious disease pathogenic bacterium in the analyte is amplified by PCR and provided as a sample to be reacted with a probe carrier, a primer set for detecting the infectious disease pathogenic bacterium can be used. The primer set suitably includes at least one selected from oligonucleotides represented in the following items (1) to (21) and at least one selected from oligonucleotides represented in the following items (22) to (28), more suitably includes all the oligonucleotides represented in the following items (1) to (28):
(1) an oligonucleotide having a base sequence of 5' gcggcgtgcctaatacatgcaag 3' (SEQ ID NO: 1);
(2) an oligonucleotide having a base sequence of 5' gcggcaggcctaacacatgcaag 3' (SEQ ID NO: 2);
(3) an oligonucleotide having a base sequence of 5' gcggcaggcttaacacatgcaag 3' (SEQ ID NO: 3);
(4) an oligonucleotide having a base sequence of 5' gcggtaggcctaacacatgcaag 3' (SEQ ID NO: 4);
(5) an oligonucleotide having a base sequence of 5' gcggcgtgcttaacacatgcaag 3' (SEQ ID NO: 5);
(6) an oligonucleotide having a base sequence of 5' gcgggatgccttacacatgcaag 3' (SEQ ID NO: 6);
(7) an oligonucleotide having a base sequence of 5' gcggcatgccttacacatgcaag 3' (SEQ ID NO: 7);
(8) an oligonucleotide having a base sequence of 5' gcggcatgcttaacacatgcaag 3' (SEQ ID NO: 8);
(9) an oligonucleotide having a base sequence of 5' gcggcgtgcttaatacatgcaag 3' (SEQ ID NO: 9);
(10) an oligonucleotide having a base sequence of 5' gcggcaggcctaatacatgcaag 3' (SEQ ID NO: 10);
(11) an oligonucleotide having a base sequence of 5' gcgggatgctttacacatgcaag 3' (SEQ ID NO: 11);
(12) an oligonucleotide having a base sequence of 5' gcggcgtgcctaacacatgcaag 3' (SEQ ID NO: 12);
(13) an oligonucleotide having a base sequence of 5' gcggcgtgcataacacatgcaag 3' (SEQ ID NO: 13);
(14) an oligonucleotide having a base sequence of 5' gcggcatgcctaacacatgcaag 3' (SEQ ID NO: 14);
(15) an oligonucleotide having a base sequence of 5' gcggcgcgcctaacacatgcaag 3' (SEQ ID NO: 15);
(16) an oligonucleotide having a base sequence of 5' gcggcgcgcttaacacatgcaag 3' (SEQ ID NO: 16);
(17) an oligonucleotide having a base sequence of 5' gcgtcatgcctaacacatgcaag 3' (SEQ ID NO: 17);
(18) an oligonucleotide having a base sequence of 5' gcgataggcttaacacatgcaag 3' (SEQ ID NO: 18);
(19) an oligonucleotide having a base sequence of 5' gcgacaggcttaacacatgcaag 3' (SEQ ID NO: 19);
(20) an oligonucleotide having a base sequence of 5' gctacaggcttaacacatgcaag 3' (SEQ ID NO: 20);
(21) an oligonucleotide having a base sequence of 5' acagaatgcttaacacatgcaag 3' (SEQ ID NO: 21);
(22) an oligonucleotide having a base sequence of 5' atccagccgcaccttccgatac 3' (SEQ ID NO: 22);
(23) an oligonucleotide having a base sequence of 5' atccaaccgcaggttcccctac 3' (SEQ ID NO: 23);
(24) an oligonucleotide having a base sequence of 5' atccagccgcaggttcccctac 3' (SEQ ID NO: 24);
(25) an oligonucleotide having a base sequence of 5' atccagccgcaccttccggtac 3' (SEQ ID NO: 25);
(26) an oligonucleotide having a base sequence of 5' atccagcgccaggttcccctag 3' (SEQ ID NO: 26);
(27) an oligonucleotide having a base sequence of 5' atccagccgcaggttctcctac 3' (SEQ ID NO: 27); and
(28) an oligonucleotide having a base sequence of 5' atccagccgcacgttcccgtac 3' (SEQ ID NO: 28).

Among them, a primer designed for allowing the amplification of *Aeromonas hydrophila* is a primer set of the following:
(2) an oligonucleotide having a base sequence of 5' gcggcaggcctaacacatgcaag 3' (SEQ ID NO: 2); and
(24) an oligonucleotide having a base sequence of 5' atccagccgcaggttcccctac 3' (SEQ ID NO: 24).

For detecting *Aeromonas hydrophila,* at least such a primer may be included.

The utilities of the respective primers (1) to (28) for amplification of *Aeromonas hydrophila* (JCM 1027) can be evaluated and confirmed by comparing each sequence of SEQ ID NOs. 1 to 28 with a DNA sequence including the 16S rRNA coding region of *Aeromonas hydrophila* (SEQ ID NO. 95).

A kit for detecting the infectious disease pathogenic bacterium can be constructed using at least a probe as described above and a reagent for detecting a reaction of the probe with a nucleic acid in an analyte. The probe in the kit can preferably be provided as a probe-immobilized carrier as described above. Further, the detection reagent may contain a label to detect the reaction or a primer for carrying out amplification as a pre-treatment.

### EXAMPLES

Hereinafter, the present invention will be described in more detail with reference to examples using probes for detecting an infectious disease pathogenic bacterium to detect *Aeromonas hydrophila.*

### (Example 1)

In this example, microorganism detection using 2-step PCR will be described.

### 1. Preparation of Probe DNA

Nucleic acid sequences shown in Table 1 were designed as probes to be used for detection of *Aeromonas hydrophila.* Specifically, the following probe base sequences were selected from the genome part coding for the 16s rRNA gene of *Aeromonas hydrophila.* These probe base sequences were designed such that they could have an extremely high specificity to the bacterium, and a sufficient hybridization sensitivity could be expected without variance for the respective probe base sequences. The probe base sequences need not always completely match with those shown in Table 1. Probes having base lengths of 20 to 30 which include the base sequences shown in Table 1 can also be used, in addition to the probes having the base sequences shown in Table 1. However, it should be ensured that the other portion of the base sequence than the portion shown in Table 1 in such a probe has no effect on the detection accuracy.

**Table 1**

| Name of microorganism | | |
|---|---|---|
| Aeromonas hydrophila | | |
| Probe No. | SEQ ID NO. | Sequence |
| 101_G_AER_01 | 92 | 5' GCCTAATACGTATCAACTGTGACGTTAC 3' |
| 101_G_AER_02 | 93 | 5' GCCTAATACGTGTCAACTGTGACGTTAC 3' |

For each probe having a base sequence shown in Table 1, a thiol group was introduced, as a functional group to immobilize the probe on a DNA chip, to the 5' terminal of the nucleic acid after synthesis in accordance with a conventional method. After introduction of the functional group, purification and freeze-drying were executed. The freeze-dried probes for internal standard were stored in a freezer at -30°C.

### 2. Preparation of PCR Primers

### 2-1. Preparation of PCR Primers for Analyte Amplification

As 16S rRNA gene (target gene) amplification PCR primers for pathogenic bacterium detection, nucleic acid sequences shown in Table 2 below were designed. Specifically, primer sets which specifically amplify the genome parts coding the 16S rRNAs, i.e., primers for which the specific melting points were made uniform as far as possible at the two end portions of the 16S rRNA coding region of a base length of 1,400 to 1,700 were designed. In order to simultaneously amplify a plurality of different bacterial species listed in the following items (1) to (80), mutants, or a plurality of 16S rRNA genes on genomes, a plurality of kinds of primers were designed. Note that a primer set is not limited to the primer sets shown in Table 2 as far as the primer set is available in common to amplify almost the entire lengths of the 16S rRNA genes of the pathogenic bacteria.

**Table 2**

| Primer No | SEQ ID NO. | Sequence |
|---|---|---|
| F01 | 1 | 5' GCGGCGTGCCTAATACATGCAAG 3' |
| F02 | 2 | 5' GCGGCAGGCCTAACACATGCAAG 3' |
| F03 | 3 | 5' GCGGCAGGCTTAACACATGCAAG 3' |
| F04 | 4 | 5' GCGGTAGGCCTAACACATGCAAG 3' |
| F05 | 5 | 5' GCGGCGTGCTTAACACATGCAAG 3' |
| F06 | 6 | 5' GCGGGATGCCTTACACATGCAAG 3' |
| F07 | 7 | 5' GCGGCATGCCTTACACATGCAAG 3' |
| F08 | 8 | 5' GCGGCATGCTTAACACATGCAAG 3' |
| F09 | 9 | 5' GCGGCGTGCTTAATACATGCAAG 3' |
| F10 | 10 | 5' GCGGCAGGCCTAATACATGCAAG 3' |
| F11 | 11 | 5' GCGGGATGCTTTACACATGCAAG 3' |
| F12 | 12 | 5' GCGGCGTGCCTAACACATGCAAG 3' |
| F13 | 13 | 5' GCGGCGTGCATAACACATGCAAG 3' |
| F14 | 14 | 5' GCGGCATGCCTAACACATGCAAG 3' |
| F15 | 15 | 5' GCGGCGCGCCTAACACATGCAAG 3' |
| F16 | 16 | 5' GCGGCGCGCTTAACACATGCAAG 3' |
| F17 | 17 | 5' GCGTCATGCCTAACACATGCAAG 3' |
| F18 | 18 | 5' GCGATAGGCTTAACACATGCAAG 3' |
| F19 | 19 | 5' GCGACAGGCTTAACACATGCAAG 3' |
| F20 | 20 | 5' GCTACAGGCTTAACACATGCAAG 3' |
| F21 | 21 | 5' ACAGAATGCTTAACACATGCAAG 3' |
| R01 | 22 | 5' ATCCAGCCGCACCTTCCGATAC 3' |
| R02 | 23 | 5' ATCCAACCGCAGGTTCCCCTAC 3' |
| R03 | 24 | 5' ATCCAGCCGCAGGTTCCCCTAC 3' |
| R04 | 25 | 5' ATCCAGCCGCACCTTCCGGTAC 3' |
| R05 | 26 | 5' ATCCAGCGCCAGGTTCCCCTAG 3' |
| R06 | 27 | 5' ATCCAGCCGCAGGTTCTCCTAC 3' |
| R07 | 28 | 5' ATCCAGCCGCACGTTCCCGTAC 3' |

(1) *Staphylococcus aureus*
(2) *Staphylococcus epidermidis*
(3) *Escherichia coli*
(4) *Klebsiella pneumoniae*
(5) *Pseudomonas aeruginosa*
(6) *Serratia marcescens*
(7) *Streptococcus pneumoniae*
(8) *Haemophilus influenzae*
(9) *Enterobacter cloacae*
(10) *Enterococcus faecalis*
(11) *Staphylococcus haemolyticus*
(12) *Staphylococcus hominis*
(13) *Staphylococcus saprophyticus*
(14) *Streptococcus agalactiae*
(15) *Streptococcus mutants*
(16) *Streptococcus pyogenes*
(17) *Streptococcus sanguinis*
(18) *Enterococcus avium*
(19) *Enterococcus faecium*
(20) *Pseudomonas fluorescens*
(21) *Pseudomonas putida*
(22) *Burkholderia cepacia*
(23) *Stenotrophomonas maltophilia*
(24) *Acinetobacter baumannii*
(25) *Acinetobacter calcoaceticus*
(26) *Achromobacter xylosoxidans*
(27) *Vibrio vulnificus*
(28) *Salmonella choleraesuis*
(29) *Citrobacter freundii*
(30) *Klebsiella oxytoca*
(31) *Enterobacter aerogenes*
(32) *Hafnia alvei*
(33) *Serratia liquefaciens*
(34) *Proteus mirabilis*
(35) *Proteus vulgaris*
(36) *Morganella morganii*
(37) *Providencia rettgeri*
(38) *Aeromonas hydrophila*
(39) *Aeromonas sobria*
(40) *Gardnerella vaginalis*
(41) *Corynebacterium diphtheriae*
(42) *Legionella pneumophila*
(43) *Bacillus cereus*
(44) *Bacillus subtilis*
(45) *Mycobacterium kansasii*
(46) *Mycobacterium intracellulare*
(47) *Mycobacterium chelonae*
(48) *Nocardia asteroides*
(49) *Bacteroides fragilis*
(50) *Bacteroides thetaiotaomicron*
(51) *Clostridium difficile*
(52) *Clostridium perfringens*
(53) *Eggerthella lenta*
(54) *Fusobacterium necrophorum*
(55) *Fusobacterium nucleatum*
(56) *Lactobacillus acidophilus*
(57) *Anaerococcus prevotii*
(58) *Peptoniphilus asaccharolyticus*
(59) *Porphyromonas asaccharolytica*
(60) *Porphyromonas gingivalis*
(61) *Prevotella denticola*
(62) *Propionibacterium acnes*
(63) *Acinetobacter johnsonii*
(64) *Acinetobacter junii*
(65) *Aeromonas schubertii*
(66) *Aeromonas veronii*
(67) *Bacteroides distasonis*
(68) *Bacteroides vulgatus*
(69) *Campylobacter coli*
(70) *Campylobacter hyointestinalis*
(71) *Campylobacter jejuni*
(72) *Flavobacterium aquatile*
(73) *Flavobacterium mizutaii*
(74) *Peptococcus niger*
(75) *Propionibacterium avidum*
(76) *Propionibacterium freudenreichii*
(77) *Propionibacterium granulosum*
(78) *Clostridium butyricum*
(79) *Flavobacterium hydatis*
(80) *Flavobacterium johnsoniae*

The primers shown in Table 2 were purified by high performance liquid chromatography (HPLC) after synthesis. The twenty-one forward primers and the seven reverse primers were mixed and dissolved in a TE buffer solution such that each primer concentration had an ultimate concentration of 10 pmol/µl.

### 2-2. Preparation of Labeling PCR Primers

In a manner similar to the above-mentioned analyte amplification primers, oligonucleotides having sequences as shown in Table 3 below were employed as primers for labeling.

**Table 3**

| Primer No | SEQ ID NO. | Sequence |
|---|---|---|
| Cy3R9-1 | 29 | 5' TACCTTGTTACGACTTCACCCCA 3' |
| Cy3R9-2 | 30 | 5' TACCTTGTTACGACTTCGTCCCA 3' |
| Cy3R9-3 | 31 | 5' TACCTTGTTACGACTTAGTCCCA 3' |
| Cy3R9-4 | 32 | 5' TACCTTGTTACGACTTAGCCCCA 3' |
| Cy3R9-5 | 33 | 5' TACCTTGTTACGACTTAGTCCTA 3' |
| Cy3R9-6 | 34 | 5' TACCTTGTTACGACTTAGCCCTA 3' |

The primers shown in Table 3 were labeled with a fluorescent dye, Cy3. The primers were purified by high performance liquid chromatography (HPLC) after synthesis. The six labeled primers were mixed and dissolved in a TE buffer solution such that each primer concentration had an ultimate concentration of 10 pmol/µl.

### 3. Extraction of Genome DNA (Model Analyte) of Aeromonas hydrophila

### 3-1. Microbial Culture & Genome DNA Extraction

First, *Aeromonas hydrophila* (JCM 1027) was cultured in accordance with the conventional method. This microbial culture medium was subjected to the extraction and purification of genome DNA by using a nucleic acid purification kit (FastPrep FP100A FastDNA Kit, manufactured by Funakoshi Co., Ltd.).

### 3-2. Test of Collected Genome DNA

The collected genome DNA of the microorganism, *Aeromonas hydrophila,* was subjected to agarose electrophoresis and 260/280-nm absorbance determination in accordance with the conventional method. Thus, the quality (the admixture amount of low molecular nucleic acid and the degree of decomposition) and the collection amount were tested. In this example, about 10 µg of the genome DNA was collected. No degradation of genome DNA or contamination of rRNA was observed. The collected genome DNA was dissolved in a TE buffer solution at an ultimate concentration of 50 ng/µl and used in the following experiments.

### 4. Preparation of DNA Chip

### 4-1. Cleaning of Glass Substrate

A glass substrate (size: 25 mm × 75 mm × 1 mm, available from Iiyama Precision Glass) made of synthetic quartz was placed in a heat- and alkali-resisting rack and dipped in a cleaning solution for ultrasonic cleaning, which was adjusted to have a predetermined concentration. The glass substrate was kept dipped in the cleaning solution for a night and cleaned by ultrasonic cleaning for 20 min. The substrate was picked up, lightly rinsed with pure water, and cleaned by ultrasonic cleaning in ultrapure water for 20 min. The substrate was dipped in a 1N aqueous sodium hydroxide solution heated to 80°C for 10 min. Pure water cleaning and ultrapure water cleaning were executed again. A quartz glass substrate for a DNA chip was thus prepared.

### 4-2. Surface Treatment

A silane coupling agent KBM-603 (available from Shin-Etsu Silicone) was dissolved in pure water at a concentration of 1% by weight (wt%) and stirred at room temperature for 2 hrs. The cleaned glass substrate was dipped in the aqueous solution of the silane coupling agent and left stand still at room temperature for 20 min. The glass substrate was picked up. The surface thereof was lightly rinsed with pure water and dried by spraying nitrogen gas to both surfaces of the substrate. The dried substrate was baked in an oven at 120°C for 1 hr to complete the coupling agent treatment, whereby an amino group was introduced to the substrate surface. Next, N-maleimidocaproyloxy succinimido (abbreviated as EMCS hereinafter) was dissolved in a 1:1 (volume ratio) solvent mixture of dimethyl sulfoxide and ethanol to obtain an ultimate concentration of 0.3 mg/ml. As a result, an EMCS solution was prepared. Here, EMCS is N-(6-maleimidocaproyloxy)succinimido available from Dojindo.

The baked glass substrate was left stand and cooled and dipped in the prepared EMCS solution at room temperature for 2 hrs. By this treatment, the amino group introduced to the surface of the substrate by the silane coupling agent reacted with the succinimide group in the EMCS to introduce the maleimide group to the surface of the glass substrate. The glass substrate picked up from the EMCS solution was cleaned by using the above-described solvent mixture in which the EMCS was dissolved. The glass substrate was further cleaned by ethanol and dried in a nitrogen gas atmosphere.

### 4-3. Probe DNA

The microorganism detection probe prepared in the stage 1 (Preparation of Probe DNA) of Example 1 was dissolved in pure water. The solution was dispensed such that the ultimate concentration (at ink dissolution) became 10 µM. Then, the solution was freeze-dried to remove water.

### 4-4. DNA Discharge by BJ Printer and Bonding to Substrate

An aqueous solution containing 7.5-wt% glycerin, 7.5-wt% thiodiglycol, 7.5-wt% urea, and 1.0-wt% Acetylenol EH (available from Kawaken Fine Chemicals) was prepared. Each of the two probes (Table 1) prepared in advance was dissolved in the solvent mixture at a specific concentration. An ink tank for an inkjet printer (trade name: BJF-850, available from Canon) is filled with the resultant DNA solution and attached to the printhead.

The inkjet printer used here was modified in advance to allow printing on a flat plate. When a printing pattern is input in accordance with a predetermined file creation method, about 5-picoliter of a DNA solution can be spotted at a pitch of about 120 µm.

The printing operation was executed for one glass substrate by using the modified inkjet printer to prepare an array. After confirming that printing was reliably executed, the glass substrate was left stand still in a humidified chamber for 30 min to make the maleimide group on the glass substrate surface react with the thiol group at the nucleic acid probe terminal.

### 4-5. Cleaning

After reaction for 30 min, the DNA solution remaining on the surface was cleaned by using a 10-mM phosphate buffer (pH 7.0) containing 100-mM NaCl, thereby obtaining a DNA chip in which single-stranded DNAs were immobilized on the glass substrate surface.

### 5. Amplification and Labeling of Analyte

### 5-1. Amplification of Analyte: 1st PCR

The amplification reaction (1st PCR) and the labeling reaction (2nd PCR) of a microbial gene to be provided as an analyte are shown in Table 4 below.

**Table 4**

| | |
|---|---|
| AmpliTaq Gold LD (5.0 U/µL) | 0.5 µL (2.5 unit/tube) |
| Primer mix <FR21×7> | 2.0 µL |
| Forward primer (×21 [0.625 µM/each]) | (final 1.25 pmol each/tube) |
| | |
| Reverse primer (×07 [1.875 µM/each]) | (final 3.75 pmol each/tube) |
| | |
| 10× PCR buffer | 5.0 µL (final 1× conc.) |
| MgCl₂ (25 mM) | 8.0 µL (final 4.0 mM) |
| dNTPmix (2.5 mM/each) | 4.0 µL (final 200 µM each) |
| Template | variable |
| H₂O | up to 50 µL |
| Total | 50 µL |

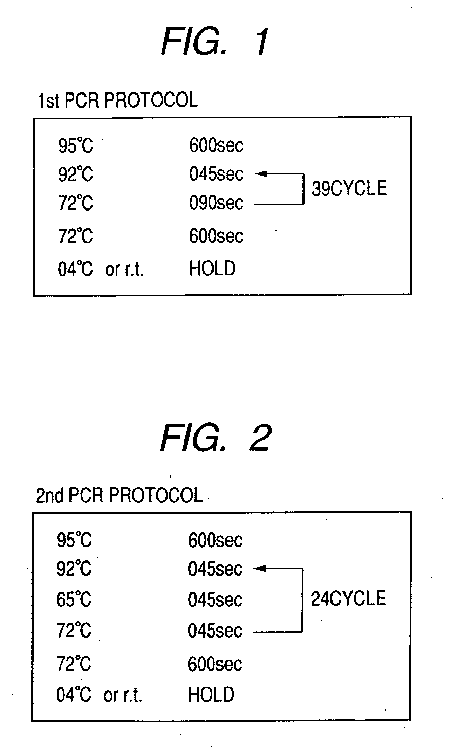

Amplification reaction of the reaction solution having the above-mentioned composition was carried out using a commercially available thermal cycler in accordance with the protocol illustrated in FIG. 1. After the end of reaction, the primer was purified using a purification column (QIAquick PCR Purification Kit available from QIAGEN). Subsequently, the quantitative assay of the amplified product was carried out.

### 5-2. Labeling Reaction: 2nd PCR

Amplification reaction of the reaction solution having the composition shown in Table 5 was carried out using a commercially available thermal cycler in accordance with the protocol illustrated in FIG. 2.

**Table 5**

| | |
|---|---|
| Premix Taq (Ex Taq Version) | 25 µL |
| Cy3-labeled reverse primer mix | 0.83 µL |
| Cy3R9 mix (×06[10 µM/each]) | (final 8.3 pmol each/tube) |
| Template | variable (final 30 ng/tube) |
| H₂O | up to 50 µL |
| Total | 50 µL |

After the end of reaction, the primer was purified using a purification column (QIAquick PCR Purification Kit available from QIAGEN) to obtain a labeled analyte.

### 6. Hybridization

Detection reaction was performed using the DNA chip prepared in the stage 4 (Preparation of DNA Chip) and the labeled analyte prepared in the stage 5 (Amplification and Labeling of Analyte).

### 6-1. Blocking of DNA Chip

Bovine serum albumin (BSA, Fraction V: available from Sigma) was dissolved in a 100-mM NaCl/10-mM phosphate buffer such that a 1 wt % solution was obtained. Then, the DNA chip prepared in the stage 4 (Preparation of DNA Chip) was dipped in the solution at room temperature for 2 hrs to execute blocking. After the end of blocking, the chip was cleaned using a washing solution as described below, rinsed with pure water and hydro-extracted by a spin dryer.

The washing solution: 2 × SSC solution (NaCl 300 mM, sodium citrate (trisodium citrate dihydrate, C₆H₅Na₃•2H₂O) 30 mM, pH 7.0) containing 0.1-wt% sodium dodecyl sulfate (SDS)

### 6-2. Hybridization

The hydro-extracted DNA chip was placed in a hybridization apparatus (Hybridization Station available from Genomic Solutions Inc). Hybridization reaction was carried out in a hybridization solution under conditions as described below.

### 6-3. Hybridization Solution

6 × SSPE/10% formamide/target (all 2nd PCR product)/0.05 wt% (6 × SSPE: NaCl 900 mM, NaH₂PO₄H₂O 50 mM, EDTA 6 mM, pH, 7.4)

### 6-4. Hybridization Conditions

65°C for 3 min, 55°C for 4 hrs, washing with 2 × SSC/0.1% SDS at 50°C, washing with 2 × SSC/0.1% SDS at 20°C (rinse with H₂O: manual), and spin dry.

### 7. Microorganism Genome Detection (Fluorometry)

The DNA chip after the end of hybridization reaction was subjected to fluorometry with a DNA chip fluorescent detector (GenePix 4000B available from Axon). As a result, *Aeromonas hydrophila* was able to be detected with a sufficient signal at a high reproducibility. The results of fluorometry are shown in Table 6 below.

**Table 6**

| Probe No. | Fluorescence intensity |
|---|---|
| | Aeromonas hydrophila (JCM 1027) |
| 038_A_hyd_01_01 | 3268.2 |
| 038_A_hyd_01_02 | 941.7 |

### 8. Hybridization with Other Bacterial Species

For proving the fact that the probe set shown in Table 1 can be specifically hybridized only with *Aeromonas hydrophila,* the results of hybridization reaction with *Aeromonas sobria* (JCM 2139) are shown in Table 7 below.

**Table 7**

| Probe No. | Fluorescence intensity |
|---|---|
| | Aeromonas sobria (JCM 2139) |
| 038_A_hyd_01_01 | 49.8 |
| 038_A_hyd_01_02 | 48.7 |

### 9. Results

As is evident from the above description, a DNA chip was prepared such that a probe set, which was able to detect only *Aeromonas hydrophila* in a specific manner, was immobilized. Further, the use of such a DNA chip allowed the identification of an infectious disease pathogenic bacterium, so the problems of the DNA probe derived from a microorganism was able to be solved. In other words, the oligonucleotide probe can be chemically produced in large amounts, while the purification or concentration thereof can be controlled. In addition, for classification of microbial species, a probe set capable of collectively detecting bacterial strains of the same genus and differentially detecting them from bacteria of other genera, was able to be provided.

Further, in addition to *Aeromonas sobria* as described above, hybridization reaction was carried out on each of nucleic acids extracted from the bacteria represented in the above-mentioned items (1) to (80). The results thereof confirmed that no substantial reaction was observed with respect to each of those bacteria in a manner similar to that of *Aeromonas sobria,* except of *Aeromonas hydrophila.*

The bacteria represented in the above-mentioned items (1) to (80) are pathogenic bacteria for septicemia, and they cover almost all of the pathogenic bacteria ever detected in human blood. Therefore, by using the primer of the present embodiment, the nucleic acid of an infectious disease pathogenic bacterium in blood can be extracted and then subjected to hybridization reaction with the probe of the present invention, whereby identification of *Aeromonas hydrophila* can be performed with higher accuracy.

Further, according to the above-mentioned example, the presence of an infectious disease pathogenic bacterium can be efficiently determined with high accuracy by completely detecting the 16S rRNA gene from the gene of the infectious disease pathogenic bacterium.

### (Example 2) Preparation of DNA Chip by which various bacterial species can be simultaneously determined

In a manner similar to the stage 1 (Preparation of Probe DNA) of Example 1, probes having base sequences as shown in Table 8 below were prepared.

**Table 8**

| Bacterial species (or genus) of interest | Probe sequence (5'→3') | SEQ ID NO. |
|---|---|---|
| *Anaerococcus prevotii* | TCATCTTGAGGTATGGAAGGGAAAGTGG | 35 |
| | GTGTTAGGTGTCTGGAATAATCTGGGTG | 36 |
| | ACCAAGTCTTGACATATTACGGCGG | 37 |
| *Bacteroides fragilis* | AAGGATTCCGGTAAAGGATGGGGATG | 38 |
| | TGGAAACATGTCAGTGAGCAATCACC | 39 |
| *Bacteroides thetaiotaomicron* | AAGAATTTCGGTTATCGATGGGGATGC | 40 |
| | AAGTTTTCCACGTGTGGAATTTTGTATGT | 41 |
| | AAGGCAGCTACCTGGTGACAGGAT | 42 |
| *Clostridium difficile* | AATATCAAAGGTGAGCCAGTACAGGATGGA | 43 |
| | CCGTAGTAAGCTCTTGAAACTGGGAGAC | 44 |
| | TCCCAATGACATCTCCTTAATCGGAGAG | 45 |
| *Clostridium perfringens* | AACCAAAGGAGCAATCCGCTATGAGAT | 46 |
| | GAGCGTAGGCGGATGATTAAGTGGG | 47 |
| | CCCTTGCATTACTCTTAATCGAGGAAATC | 48 |
| *Eggerthella lenta* | GGAAAGCCCAGACGGCAAGGGA | 49 |
| | CCTCTCAAGCGGGATCTCTAATCCGA | 50 |
| | TGCCCCATGTTGCCAGCATTAGG | 51 |
| *Fusobacterium necrophorum* | TTTTCGCATGGAGGAATCATGAAAGCTA | 52 |
| | AGATGCGCCGGTGCCCTTTCG | 53 |
| | AGTCGGGAAGAAGTCAGTGACGGTAC | 54 |
| *Peptoniphilus asaccharolyticus* | GAGTACGTGCGCAAGCATGAAACT | 55 |
| *Porphyromonas asaccharolytica* | GAAGACTGCCCGCAAGGGTTGTAA | 56 |
| | GTGTACTGGAGGTACGTGGAACGTG | 57 |
| | GCATGAGGCTGAGAGGTCTCTTCC | 58 |
| *Porphyromonas gingivalis* | TTATAGCTGTAAGATAGGCATGCGTCCC | 59 |
| | AACGGGCGATACGAGTATTGCATTGA | 60 |
| | ATATACCGTCAAGCTTCCACAGCGA | 61 |
| *Enterococcus avium* | TTTGAAAGGCGCTTTTGCGTCACTG | 62 |
| | CAAGGATGAGAGTAGAACGTTCATCCCTTG | 63 |
| | CAAGGATGAGAGTAGAATGTTCATCCCTTG | 64 |
| *Providencia rettgeri* | CCTGGGAATGGCATCTAAGACTGGTCA | 65 |
| | GAGGAAGGCGTTGATGCTAATATCATCA | 66 |
| | GAGCAAAGCAGGGGAACTTCGGTC | 67 |
| *Acinetobacter(genus)* | GTTGGGGCCTTTGAGGCTTTAGTG | 68 |
| | TGGGAGAGGATGGTAGAATTCCAGGT | 69 |
| *Prevotella denticola* | TCGATGACGGCATCAGATTCGAAGCA | 70 |
| | AATGTAGGCGCCCAACGTCTGACT | 71 |
| | ATGTTGAGGTCCTTCGGGACTCCT | 72 |
| *Flavobacterium (genus)* | GGAAGTAACTAGAATATGTAGTGTAGCGGTG | 73 |
| | GCCAGTGCAAACTGTGAGGAAGGT | 74 |
| | GGGTAGGGGTCCTGAGAGGGAGATC | 75 |
| *Aeromonas (genus)* | GAGTGCCTTCGGGAATCAGAACAC | 76 |
| | CTGCAAGCTAGCGATAGTGAGCGA | 77 |
| *Bacteroides (genus)* | CGATGGATAGGGGTTCTGAGAGGAA | 78 |
| | TGCGGCTCAACCGTAAAATTGCAGT | 79 |
| | TGTGGCTCAACCATAGAATTGCCGT | 80 |
| *Peptococcus niger* | GTACCTGTAAGAAAGACGGCCTTCGT | 81 |
| | CTGCCGAGTGATGTAATGTCACTTTTC | 82 |
| | TCGGAGGTTTCAAGACCGTCGG | 83 |
| *Clostridium(genus)* | ACCAAAGGAGCAATCCGCTATGAGATG | 84 |
| | ATCAAAGGTGAGCCAGTACAGGATGG | 85 |
| | ATTAAAGGAGTAATCCGCTATGAGATGGACC | 86 |
| *Propionibacterium acnes* | GGGCTAATACCGGATAGGAGCTCCTG | 87 |
| | AAGCGTGAGTGACGGTAATGGGTAAA | 88 |
| | ATCGCGTCGGAAGTGTAATCTTGGG | 89 |
| *Campylobacter(genus)* | TGGAGCAAATCTATAAAATATGTCCCAGT | 90 |
| | ACAGTGGAATCAGCGACTGGGG | 91 |
| *Aeromonas hydrophila* | GCCTAATACGTATCAACTGTGACGTTAC | 92 |
| | GCCTAATACGTGTCAACTGTGACGTTAC | 93 |
| *Propioniba cterium (genus)* | GCTTTCGATACGGGTTGACTTGAGGAA | 94 |

Those probes are capable of specifically detecting certain bacterial species (or genera) shown in the left column in the table just as one specific to *Aeromonas hydrophila* of Example 1.

Further, those probes are designed such that they have the same Tm value as that of a target, the same reactivity with a non-target sequence, and the like so that the nucleic acid of the bacterial species of interest can be specifically detected under the same reaction conditions.

For the respective probes, probe solutions were prepared in a manner similar to the stage 4-3 of Example 1. Subsequently, the inkjet printer used in the stage 4-4 of Example 1 was employed to discharge each of the probe solution on the same substrate to form a plurality of DNA chips having spots of the respective probes being arranged at a pitch of about 120 µm.

One of the DNA chips was used for hybridization with the nucleic acid extracted from *Aeromonas hydrophila* in a manner similar to the stage 6 of Example 1. As a result, the spot of the probe which specifically detected *Aeromonas hydrophila* showed almost the equal fluorescence intensity as that of Example 1. In contrast, the spots of other probes showed extremely low fluorescence intensity.

Further, other prepared DNA chips were used for hybridization with the bacteria listed in Table 8 except of *Aeromonas hydrophila.* As a result, the spot of *Aeromonas hydrophila* showed extremely low fluorescence intensity, while the spot of the probe for the bacterial species of interest showed extremely high fluorescence intensity. Therefore, the DNA chip prepared in the present example was confirmed that it was able to simultaneously determine 15 bacterial species and 7 genera listed in Table 8 in addition to *Aeromonas hydrophila.* By simultaneously using probes for a target species and the corresponding genus (for example, *Propionibacterium* (genus) and *Propionibactrium acnes*)*,* highly accurate identification of the target species or simultaneous identification of a plurality of target species of the same genus can be performed.

### (Example 3)

Using the DNA chip prepared in Example 2, detection was attempted when a plurality of bacterial species was present in an analyte.

A culture medium in which *Aeromonas hydrophila* and *Eggerthella lenta* were cultured was prepared and subjected to the same treatment as that of Example 1 to react with the DNA chip.

As a result, only the spots of the probes having SEQ ID NOS. 49, 50, 51, 76, 77, 92, and 93 showed high fluorescence intensity, so the coexistence of those bacteria was able to be simultaneously confirmed.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. A probe-immobilized carrier for detecting a gene of infectious disease pathogenic bacterium, *Aeromonas hydrophila,* wherein at least one first probe is arranged on a solid-phase carrier and the base sequence of said first probe is any one of the following base sequences (1) to (3):
(1) GCCTAATACGTATCAACTGTGACGTTAC (SEQ ID NO. 92) or the complementary sequence thereof;
(2) GCCTAATACGTGTCAACTGTGACGTTAC (SEQ ID NO. 93) or the complementary sequence thereof; and
(3) a modified sequence of a length of 20 to 30 nucleotides and prepared such that any one of the sequences of SEQ ID NOS. 92 to 93 and the complementary sequences thereof is subjected to base deletion, substitution, or addition as far as 80% or more of the base sequence is consecutively conserved and as far as the modified sequences hybridize with said bacterium under the following stringent conditions: Hybridization Solution: 6 x SSPE/10% formamide/target/0.05 wt% (6 x SSPE: NaCl 900 mM, NaH₂PO₄H₂O 50 mM, EDTA 6 mM, pH, 7.4); Hybridization Conditions: 65°C for 3 min, 55°C for 4 hrs, washing with 2 x SSC/0.1% SDS at 50°C, washing with 2 x SSC/0.1% SDS at 20°C; and
wherein the probe-immobilized carrier comprises at least one second probe with the base sequence being any one of the base sequences of SEQ ID NOS. 35-91,94 as mentioned in the specification immobilized at a position spaced from the first probe.

2. The probe-immobilized carrier according to claim 1, wherein for each of said base sequences SEQ ID NOS. 92 to 93, the melting temperature between the probe sequence and the corresponding sequence of the gene of said infectious bacterium under said stringent conditions is by at least 10°C higher than the melting temperature between the same probe sequence and any gene sequence of any other infectious bacterium selected from the group of 80 bacteria (1) to (80) listed in Table 2 of the description under said stringent conditions.

3. A kit for detecting a gene of *Aeromonas hydrophila,* comprising:
a probe-immobilized carrier according to claim 1; and
a reagent for detecting a reaction between the probe and a target nucleic acid.

4. A kit according to claim 3, wherein the reagent contains a primer for amplifying the gene of *Aeromonas hydrophila,* and the primer includes:
(2) an oligonucleotide having a base sequence of 5' gcggcaggcctaacacatgcaag 3' (SEQ ID NO: 2); and
(24) an oligonucleotide having a base sequence of 5' atccagccgcaggttcccctac 3' (SEQ ID NO: 24).

5. A gene detection kit, comprising:
a probe-immobilized carrier according to claim 1; and
a reagent for detecting a reaction between the probes and a target nucleic acid,
wherein the reagent contains a set of primers including at least one oligonucleotide selected from the following items (1) to (21) and at least one oligonucleotide selected from the following items (22) to (28):
(1) an oligonucleotide having a base sequence of 5' gcggcgtgcctaatacatgcaag 3' (SEQ ID NO: 1);
(2) an oligonucleotide having a base sequence of 5' gcggcaggcctaacacatgcaag 3' (SEQ ID NO: 2);
(3) an oligonucleotide having a base sequence of 5' gcggcaggcttaacacatgcaag 3' (SEQ ID NO: 3);
(4) an oligonucleotide having a base sequence of 5' gcggtaggcctaacacatgcaag 3' (SEQ ID NO: 4);
(5) an oligonucleotide having a base sequence of 5' gcggcgtgcttaacacatgcaag 3' (SEQ ID NO: 5);
(6) an oligonucleotide having a base sequence of 5' gcgggatgccttacacatgcaag 3' (SEQ ID NO: 6);
(7) an oligonucleotide having a base sequence of 5' gcggcatgccttacacatgcaag 3' (SEQ ID NO: 7);
(8) an oligonucleotide having a base sequence of 5' gcggcatgcttaacacatgcaag 3' (SEQ ID NO: 8);
(9) an oligonucleotide having a base sequence of 5' gcggcgtgcttaatacatgcaag 3' (SEQ ID NO: 9);
(10) an oligonucleotide having a base sequence of 5' gcggcaggcctaatacatgcaag 3' (SEQ ID NO: 10);
(11) an oligonucleotide having a base sequence of 5' gcgggatgctttacacatgcaag 3' (SEQ ID NO: 11);
(12) an oligonucleotide having a base sequence of 5' gcggcgtgcctaacacatgcaag 3' (SEQ ID NO: 12);
(13) an oligonucleotide having a base sequence of 5' gcggcgtgcataacacatgcaag 3' (SEQ ID NO: 13);
(14) an oligonucleotide having a base sequence of 5' gcggcatgcctaacacatgcaag 3' (SEQ ID NO: 14);
(15) an oligonucleotide having a base sequence of 5' gcggcgcgcctaacacatgcaag 3' (SEQ ID NO: 15);
(16) an oligonucleotide having a base sequence of 5' gcggcgcgcttaacacatgcaag 3' (SEQ ID NO: 16);
(17) an oligonucleotide having a base sequence of 5' gcgtcatgcctaacacatgcaag 3' (SEQ ID NO: 17);
(18) an oligonucleotide having a base sequence of 5' gcgataggcttaacacatgcaag 3' (SEQ ID NO: 18);
(19) an oligonucleotide having a base sequence of 5' gcgacaggcttaacacatgcaag 3' (SEQ ID NO: 19);
(20) an oligonucleotide having a base sequence of 5' gctacaggcttaacacatgcaag 3' (SEQ ID NO: 20);
(21) an oligonucleotide having a base sequence of 5' acagaatgcttaacacatgcaag 3' (SEQ ID NO: 21);
(22) an oligonucleotide having a base sequence of 5' atccagccgcaccttccgatac 3' (SEQ ID NO: 22);
(23) an oligonucleotide having a base sequence of 5' atccaaccgcaggttcccctac 3' (SEQ ID NO: 23);
(24) an oligonucleotide having a base sequence of 5' atccagccgcaggttcccctac 3' (SEQ ID NO: 24);
(25) an oligonucleotide having a base sequence of 5' atccagccgcaccttccggtac 3' (SEQ ID NO: 25);
(26) an oligonucleotide having a base sequence of 5' atccagcgccaggttcccctag 3' (SEQ ID NO: 26);
(27) an oligonucleotide having a base sequence of 5' atccagccgcaggttctcctac 3' (SEQ ID NO: 27); and
(28) an oligonucleotide having a base sequence of 5' atccagccgcacgttcccgtac 3' (SEQ ID NO: 28).

6. A probe-immobilized carrier for detecting a gene of infectious disease pathogenic bacterium, *Aeromonas hydrophila,* comprising a plurality of first probes arranged on a solid-phase carrier at intervals from each other, said plurality of probes being selected from the following items (A) to (H):
(A) a probe wherein the base sequence is represented by GCCTAATACGTATCAACTGTGACGTTAC (SEQ ID NO. 92);
(B) a probe wherein the base sequence is represented by GCCTAATACGTGTCAACTGTGACGTTAC (SEQ ID NO. 93);
(C) a probe wherein the base sequence is the complementary sequence of the base sequence represented by SEQ ID NO. 92;
(D) a probe wherein the base sequence is the complementary sequence of the base sequence represented by SEQ ID NO. 93;
(E) a probe wherein the base sequence is a modified sequence with a length of 20 to 30 nucleotides, obtained by base deletion, substitution, or addition on the base sequence represented by SEQ ID NO. 92 as far as 80% or more of the base sequence is consecutively conserved and as far as the modified sequence retains the function of a probe for detecting the gene of *Aeromonas hydrophila* under the following stringent conditions: Hybridization Solution: 6 x SSPE/10% formamide/target/0.05 wt% (6 x SSPE: NaCl 900 mM, NaH₂PO₄H₂O 50 mM, EDTA 6 mM, pH, 7.4); Hybridization Conditions: 65°C for 3 min, 55°C for 4 hrs, washing with 2 x SSC/0.1% SDS at 50°C, washing with 2 x SSC/0.1% SDS at 20°C;
(F) a probe wherein the base sequence is a modified sequence with a length of 20 to 30 nucleotides, obtained by base deletion, substitution, or addition on the base sequence represented by SEQ ID NO. 93 as far as 80% or more of the base sequence is consecutively conserved and as far as the modified sequence retains the function of a probe for detecting the gene of *Aeromonas hydrophila* under the following stringent conditions: Hybridization Solution: 6 x SSPE/10% formamide/target/0.05 wt% (6 x SSPE: NaCl 900 mM, NaH₂PO₄H₂O 50 mM, EDTA 6 mM, pH, 7.4); Hybridization Conditions: 65°C for 3 min, 55°C for 4 hrs, washing with 2 x SSC/0.1% SDS at 50°C, washing with 2 x SSC/0.1% SDS at 20°C;
(G) a probe wherein the base sequence is a modified sequence with a length of 20 to 30 nucleotides, obtained by base deletion, substitution, or addition on the complementary sequence of the base sequence represented by SEQ ID NO. 92 as far as 80% or more of the complementary sequence is consecutively conserved and as far as the modified sequence retains the function of a probe for detecting the gene of *Aeromonas hydrophila* under the following stringent conditions: Hybridization Solution: 6 x SSPE/10% formamide/target/0.05 wt% (6 x SSPE: NaCl 900 mM, NaH₂PO₄H₂O 50 mM, EDTA 6 mM, pH, 7.4); Hybridization Conditions: 65°C for 3 min, 55°C for 4 hrs, washing with 2 x SSC/0.1% SDS at 50°C, washing with 2 x SSC/0.1% SDS at 20°C; and
(H) a probe wherein the base sequence is a modified sequence with a length of 20 to 30 nucleotides, obtained by base deletion, substitution, or addition on the complementary sequence of the base sequence represented by SEQ ID NO. 93 as far as 80% or more of the complementary sequence is consecutively conserved and as far as the modified sequence retains the function of a probe for detecting the gene of *Aeromonas hydrophila* under the following stringent conditions: Hybridization Solution: 6 x SSPE/10% formamide/target/0.05 wt% (6 x SSPE: NaCl 900 mM, NaH₂PO₄H₂O 50 mM, EDTA 6 mM, pH, 7.4); Hybridization Conditions: 65°C for 3 min, 55°C for 4 hrs, washing with 2 x SSC/0.1% SDS at 50°C, washing with 2 x SSC/0.1% SDS at 20°C.; and
wherein the probe-immobilized carrier comprises at least one second probe having any one of the base sequences of SEQ ID NOs 35-91,94 as mentioned in the specification immobilized at a position spaced from the plurality of first probes.

7. A method of detecting a gene of *Aeromonas hydrophila* in an analyte by using a probe-immobilized carrier, comprising the steps of:
(i) reacting the analyte with a probe-immobilized carrier according to claim 1; and
(ii) detecting the presence or absence of a reaction of the probe on the probe-immobilized carrier with a nucleic acid in the analyte, or detecting the strength of a hybridization reaction of the probe on the probe-immobilized carrier with a nucleic acid in the analyte.

8. A method according to claim 7, further comprising the step of carrying out PCR amplification of the target nucleic acid in the analyte by using a primer including the following oligonucleotides:
(2) an oligonucleotide having a base sequence of 5' gcggcaggcctaacacatgcaag 3' (SEQ ID NO: 2); and
(24) an oligonucleotide having a base sequence of 5' atccagccgcaggttcccctac 3' (SEQ ID NO: 24).

9. A probe set for specifically detecting a gene of infectious disease pathogenic bacterium, *Aeromonas hydrophila,* comprising two probes consisting of the following base sequences (1) and (2) respectively:
(1) GCCTAATACGTATCAACTGTGACGTTAC (SEQ ID NO. 92); and
(2) GCCTAATACGTGTCAACTGTGACGTTAC (SEQ ID NO. 93).

## Patentansprüche

1. Träger mit immobilisierten Sonden für den Nachweis eines Gens eines pathogenen Bakteriums einer Infektionskrankheit, *Aeromonas hydrophila,*
bei dem mindestens eine erste Sonde auf einem Festphasenträger angeordnet ist und die Basensequenz der ersten Sonde eine der folgenden Basensequenzen (1) bis (3) ist:
(1) GCCTAATACGTATCAACTGTGACGTTAC (Seq.-Ident.-Nr. 92) oder deren Komplementärsequenz;
(2) GCCTAATACGTGTCAACTGTGACGTTAC (Seq.-Ident.-Nr. 93) oder deren Komplementärsequenz; und
(3) eine modifizierte Sequenz mit einer Länge von 20 bis 30 Nucleotiden und derart hergestellt, dass eine Sequenz aus Seq.-Ident.-Nr. 92 bis 93 und deren Komplementärsequenzen einer Basendeletion, -substitution oder -addition unterzogen wurde, und zwar soweit, dass 80% oder mehr der Basensequenz aufeinanderfolgend erhalten geblieben sind, und soweit, dass die modifizierten Sequenzen unter folgenden stringenten Bedingungen mit dem Bakterium hybridisieren: Hybridisierungslösung: 6 x SSPE/10% Formamid/Target/0,05 Gew.% (6 x SSPE: NaCl 900 mM, NaH₂PO₄H₂O 50 mM, EDTA 6 mM, pH 7,4); Hybridisierungsbedingungen: 65°C für 3 min, 55°C für 4 h, Waschen mit 2 x SSC/0,1% SDS bei 50°C, Waschen mit 2 x SSC/0,1% SDS bei 20°C; und
wobei der Träger mit immobilisierten Sonden mindestens eine zweite Sonde, deren Basensequenz eine der Basensequenzen mit Seq.-Ident.-Nr. 35-91, 94 wie in der Beschreibung aufgeführt ist, umfasst, die an einer von der ersten Sonde beabstandeten Position immobilisiert ist.

2. Träger mit immobilisierten Sonden nach Anspruch 1,
bei dem für jede der Basensequenzen mit Seq.-Ident.-Nr. 92 bis 93 die Schmelztemperatur unter diesen stringenten Bedingungen zwischen der Sondensequenz und der entsprechenden Sequenz des Gens des infektiösen Bakteriums um mindestens 10°C höher ist als die Schmelztemperatur unter diesen stringenten Bedingungen zwischen derselben Sondensequenz und irgendeiner Gensequenz irgendeines anderen infektiösen Bakteriums, das aus der Gruppe der 80 in Tabelle 2 der Beschreibung aufgeführten Bakterien (1) bis (80) ausgewählt ist.

3. Kit für den Nachweis eines Gens von *Aeromonas hydrophila,* umfassend:
einen Träger mit immobilisierten Sonden nach Anspruch 1; und
ein Reagens für den Nachweis einer Reaktion zwischen der Sonde und einer Targetnucleinsäure.

4. Kit nach Anspruch 3,
bei dem das Reagens einen Primer zum Amplifizieren des Gens von *Aeromonas hydrophila* enthält und der Primer Folgendes beinhaltet:
(2) ein Oligonucleotid mit Basensequenz 5' gcggcaggcctaacacatgcaag 3' (Seq.-Ident.-Nr. 2); und
(24) ein Oligonucleotid mit Basensequenz 5' atccagccgcaggttcccctac 3' (Seq.-Ident.-Nr. 24).

5. Kit für den Gennachweis, umfassend:
einen Träger mit immobilisierten Sonden nach Anspruch 1; und
ein Reagens für den Nachweis einer Reaktion zwischen den Sonden und einer Targetnucleinsäure,
wobei das Reagens einen Satz Primer enthält, der mindestens ein aus den folgenden Punkten (1) bis (21) ausgewähltes Oligonucleotid sowie mindestens ein aus den folgenden Punkten (22) bis (28) ausgewähltes Oligonucleotid beinhaltet:
(1) ein Oligonucleotid mit Basensequenz 5' gcggcgtgcctaatacatgcaag 3' (Seq.-Ident.-Nr. 1);
(2) ein Oligonucleotid mit Basensequenz 5' gcggcaggcctaacacatgcaag 3' (Seq.-Ident.-Nr. 2);
(3) ein Oligonucleotid mit Basensequenz 5' gcggcaggcttaacacatgcaag 3' (Seq.-Ident.-Nr. 3);
(4) ein Oligonucleotid mit Basensequenz 5' gcggtaggcctaacacatgcaag 3' (Seq.-Ident.-Nr. 4);
(5) ein Oligonucleotid mit Basensequenz 5' gcggcgtgcttaacacatgcaag 3' (Seq.-Ident.-Nr. 5);
(6) ein Oligonucleotid mit Basensequenz 5' gcgggatgccttacacatgcaag 3' (Seq.-Ident.-Nr. 6);
(7) ein Oligonucleotid mit Basensequenz 5' gcggcatgccttacacatgcaag 3' (Seq.-Ident.-Nr. 7);
(8) ein Oligonucleotid mit Basensequenz 5' gcggcatgcttaacacatgcaag 3' (Seq.-Ident.-Nr. 8);
(9) ein Oligonucleotid mit Basensequenz 5' gcggcgtgcttaatacatgcaag 3' (Seq.-Ident.-Nr. 9);
(10) ein Oligonucleotid mit Basensequenz 5' gcggcaggcctaatacatgcaag 3' (Seq.-Ident.-Nr. 10);
(11) ein Oligonucleotid mit Basensequenz 5' gcgggatgctttacacatgcaag 3' (Seq.-Ident.-Nr. 11);
(12) ein Oligonucleotid mit Basensequenz 5' gcggcgtgcctaacacatgcaag 3' (Seq.-Ident.-Nr. 12);
(13) ein Oligonucleotid mit Basensequenz 5' gcggcgtgcataacacatgcaag 3' (Seq.-Ident.-Nr. 13);
(14) ein Oligonucleotid mit Basensequenz 5' gcggcatgcctaacacatgcaag 3' (Seq.-Ident.-Nr. 14);
(15) ein Oligonucleotid mit Basensequenz 5' gcggcgcgcctaacacatgcaag 3' (Seq.-Ident.-Nr. 15);
(16) ein Oligonucleotid mit Basensequenz 5' gcggcgcgcttaacacatgcaag 3' (Seq.-Ident.-Nr. 16);
(17) ein Oligonucleotid mit Basensequenz 5' gcgtcatgcctaacacatgcaag 3' (Seq.-Ident.-Nr. 17);
(18) ein Oligonucleotid mit Basensequenz 5' gcgataggcttaacacatgcaag 3' (Seq.-Ident.-Nr. 18);
(19) ein Oligonucleotid mit Basensequenz 5' gcgacaggcttaacacatgcaag 3' (Seq.-Ident.-Nr. 19);
(20) ein Oligonucleotid mit Basensequenz 5' gctacaggcttaacacatgcaag 3' (Seq.-Ident.-Nr. 20);
(21) ein Oligonucleotid mit Basensequenz 5' acagaatgcttaacacatgcaag 3' (Seq.-Ident.-Nr. 21);
(22) ein Oligonucleotid mit Basensequenz 5' atccagccgcaccttccgatac 3' (Seq.-Ident.-Nr. 22);
(23) ein Oligonucleotid mit Basensequenz 5' atccaaccgcaggttcccctac 3' (Seq.-Ident.-Nr. 23);
(24) ein Oligonucleotid mit Basensequenz 5' atccagccgcaggttcccctac 3' (Seq.-Ident.-Nr. 24);
(25) ein Oligonucleotid mit Basensequenz 5' atccagccgcaccttccggtac 3' (Seq.-Ident.-Nr. 25);
(26) ein Oligonucleotid mit Basensequenz 5' atccagcgccaggttcccctag 3' (Seq.-Ident.-Nr. 26);
(27) ein Oligonucleotid mit Basensequenz 5' atccagccgcaggttctcctac 3' (Seq.-Ident.-Nr. 27); und
(28) ein Oligonucleotid mit Basensequenz 5' atccagccgcacgttcccgtac 3' (Seq.-Ident.-Nr. 28).

6. Träger mit immobilisierten Sonden für den Nachweis eines Gens eines pathogenen Bakteriums einer Infektionskrankheit, *Aeromonas hydrophila,*
der mehrere auf einem Festphasenträger in Abständen zueinander angeordnete erste Sonden umfasst, wobei die mehreren Sonden aus den folgenden Punkten (A) bis (H) ausgewählt sind:
(A) eine Sonde, bei der die Basensequenz durch GCCTAATACGTATCAACTGTGACGTTAC (Seq.-Ident.-Nr. 92) dargestellt ist;
(B) eine Sonde, bei der die Basensequenz durch GCCTAATACGTGTCAACTGTGACGTTAC (Seq.-Ident.-Nr. 93) dargestellt ist;
(C) eine Sonde, bei der die Basensequenz die Komplementärsequenz der durch Seq.-Ident.-Nr. 92 dargestellten Basensequenz ist;
(D) eine Sonde, bei der die Basensequenz die Komplementärsequenz der durch Seq.-Ident.-Nr. 93 dargestellten Basensequenz ist;
(E) eine Sonde, bei der die Basensequenz eine modifizierte Sequenz mit einer Länge von 20 bis 30 Nucleotiden ist, die durch Basendeletion, -substitution oder -addition an der durch Seq.-Ident.-Nr. 92 dargestellten Basensequenz erhalten wurde, und zwar soweit, dass 80% oder mehr der Basensequenz aufeinanderfolgend erhalten geblieben sind, und soweit, dass die modifizierte Sequenz die Funktion als Sonde für den Nachweis des Gens von *Aeromonas hydrophila* unter den folgenden stringenten Bedingungen beibehält:
Hybridisierungslösung: 6 x SSPE/10% Formamid/Target/0,05 Gew.% (6 x SSPE: NaCl 900 mM, NaH₂PO₄H₂O 50 mM, EDTA 6 mM, pH 7,4); Hybridisierungsbedingungen: 65°C für 3 min, 55°C für 4 h, Waschen mit 2 x SSC/0,1% SDS bei 50°C, Waschen mit 2 x SSC/0,1% SDS bei 20°C;
(F) eine Sonde, bei der die Basensequenz eine modifizierte Sequenz mit einer Länge von 20 bis 30 Nucleotiden ist, die durch Basendeletion, -substitution oder -addition an der durch Seq.-Ident.-Nr. 93 dargestellten Basensequenz erhalten wurde, und zwar soweit, dass 80% oder mehr der Basensequenz aufeinanderfolgend erhalten geblieben sind, und soweit, dass die modifizierte Sequenz die Funktion als Sonde für den Nachweis des Gens von *Aeromonas hydrophila* unter den folgenden stringenten Bedingungen beibehält:
Hybridisierungslösung: 6 x SSPE/10% Formamid/Target/0,05 Gew.% (6 x SSPE: NaCl 900 mM, NaH₂PO₄H₂O 50 mM, EDTA 6 mM, pH 7,4); Hybridisierungsbedingungen: 65°C für 3 min, 55°C für 4 h, Waschen mit 2 x SSC/0,1% SDS bei 50°C, Waschen mit 2 x SSC/0,1% SDS bei 20°C;
(G) eine Sonde, bei der die Basensequenz eine modifizierte Sequenz mit einer Länge von 20 bis 30 Nucleotiden ist, die durch Basendeletion, -substitution oder -addition an der Komplementärsequenz der durch Seq.-Ident.-Nr. 92 dargestellten Basensequenz erhalten wurde, und zwar soweit, dass 80% oder mehr der Komplementärsequenz aufeinanderfolgend erhalten geblieben sind, und soweit, dass die modifizierte Sequenz die Funktion als Sonde für den Nachweis des Gens von *Aeromonas hydrophila* unter den folgenden stringenten Bedingungen beibehält:
Hybridisierungslösung: 6 x SSPE/10% Formamid/Target/0,05 Gew.% (6 x SSPE: NaCl 900 mM, NaH₂PO₄H₂O 50 mM, EDTA 6 mM, pH 7,4); Hybridisierungsbedingungen: 65°C für 3 min, 55°C für 4 h, Waschen mit 2 x SSC/0,1 % SDS bei 50°C, Waschen mit 2 x SSC/0,1% SDS bei 20°C; und
(H) eine Sonde, bei der die Basensequenz eine modifizierte Sequenz mit einer Länge von 20 bis 30 Nucleotiden ist, die durch Basendeletion, -substitution oder -addition an der Komplementärsequenz der durch Seq.-Ident.-Nr. 93 dargestellten Basensequenz erhalten wurde, und zwar soweit, dass 80% oder mehr der Komplementärsequenz aufeinanderfolgend erhalten geblieben sind, und soweit, dass die modifizierte Sequenz die Funktion als Sonde für den Nachweis des Gens von *Aeromonas hydrophila* unter den folgenden stringenten Bedingungen beibehält:
Hybridisierungslösung: 6 x SSPE/10% Formamid/Target/0,05 Gew. % (6 x SSPE: NaCl 900 mM, NaH₂PO₄H₂O 50 mM, EDTA 6 mM, pH 7,4); Hybridisierungsbedingungen: 65°C für 3 min, 55°C für 4 h, Waschen mit 2 x SSC/0,1% SDS bei 50°C, Waschen mit 2 x SSC/0,1% SDS bei 20°C.; und
wobei der Träger mit immobilisierten Sonden mindestens eine zweite Sonde mit einer der Basensequenzen mit Seq.-Ident.-Nr. 35-91, 94 wie in der Beschreibung aufgeführt umfasst, die an einer von den mehreren ersten Sonden beabstandeten Position immobilisiert ist.

7. Verfahren zum Nachweis eines Gens von *Aeromonas hydrophila* in einem Analyt unter Verwendung eines Trägers mit immobilisierten Sonden, das die folgenden Schritte umfasst:
(i) Reagieren des Analyts mit einem Träger mit immobilisierten Sonden nach Anspruch 1; und
(ii) Nachweis des Vorhandenseins oder Fehlens einer Reaktion der Sonde auf dem Träger mit immobilisierten Sonden mit einer Nucleinsäure im Analyt, oder Ermitteln der Stärke einer Hybridisierungsreaktion der Sonde auf dem Träger mit immobilisierten Sonden mit einer Nucleinsäure im Analyt.

8. Verfahren nach Anspruch 7,
weiterhin umfassend den Schritt zum Durchführen von PCR-Amplifikation der Targetnucleinsäure im Analyt unter Verwendung eines die folgenden Oligonucleotide beinhaltenden Primers:
(2) ein Oligonucleotid mit Basensequenz 5' gcggcaggcctaacacatgcaag 3' (Seq.-Ident.-Nr. 2); und
(24) ein Oligonucleotid mit Basensequenz 5' atccagccgcaggttcccctac 3' (Seq.-Ident.-Nr. 24).

9. Sondensatz für den spezifischen Nachweis eines Gens eines pathogenen Bakteriums einer Infektionskrankheit, *Aeromonas hydrophila,* der zwei aus den folgenden Basensequenzen (1) bzw. (2) bestehende Sonden umfasst:
(1) GCCTAATACGTATCAACTGTGACGTTAC (Seq.-Ident.-Nr. 92); und
(2) GCCTAATACGTGTCAACTGTGACGTTAC (Seq.-Ident.-Nr. 93).

## Revendications

1. Support à sondes immobilisées pour détecter un gène d'une bactérie pathogène de maladie infectieuse, *Aeromonas hydrophila,* dans lequel au moins une première sonde est disposée sur un support en phase solide et la séquence de bases de ladite première sonde est l'une quelconque des séquences de bases (1) à (3) suivantes :
(1) GCCTAATACGTATCAACTGTGACGTTAC (SEQ ID N° 92) ou sa séquence complémentaire ;
(2) GCCTAATACGTGTCAACTGTGACGTTAC (SEQ ID N° 93) ou sa séquence complémentaire ; et
(3) une séquence modifiée d'une longueur de 20 à 30 nucléotides et préparée de telle sorte que l'une quelconque de la séquence des SEQ ID N° 92 et 93 et leurs séquences complémentaires soit soumise à une délétion, substitution ou addition de bases dans la mesure où 80 % ou plus de la séquence de bases sont conservés de manière consécutive et dans la mesure où la séquence modifiée s'hybride avec ladite bactérie dans les conditions drastiques suivantes : solution d'hybridation : SSPE 6 x/10 % de formamide/cible/ 0,05 % en poids (SSPE 6 x : NaCl 900 mM, NaH₂PO₄H₂O 50 mM, EDTA 6 mM, pH 7,4) ; conditions d'hybridation : 65°C pendant 3 min, 55°C pendant 4 h, lavage avec SSC 2 x/0,1 % de SDS à 50°C, lavage avec SSC 2 x/0,1 % de SDS à 20°C ; et
ledit support à sondes immobilisées comprenant au moins une seconde sonde dont la séquence de bases est l'une quelconque des séquence de bases des SEQ ID N°^{s} 35-91, 94 mentionnées dans la description immobilisée à une position espacée de la première sonde.

2. Support à sondes immobilisées suivant la revendication 1, dans lequel, pour chacune desdites séquences de bases SEQ ID N°^{s} 92 et 93, la température de fusion entre la séquence de sonde et la séquence correspondante du gène de ladite bactérie infectieuse dans lesdites conditions drastiques est supérieure d'au moins 10°C à la température de fusion entre la même séquence de sonde et n'importe quelle séquence de gène de n'importe quelle autre bactérie infectieuse choisie dans le groupe des 80 bactéries (1) à (80) énumérées sur le tableau 2 de la description dans lesdites conditions drastiques.

3. Kit pour détecter un gène *d'Aeromonas hydrophila,* comprenant :
un support à sondes immobilisées suivant la revendication 1 ; et
un réactif pour détecter une réaction entre la sonde et un acide nucléique cible.

4. Kit suivant la revendication 3, dans lequel le réactif contient une amorce pour amplifier le gène *d'Aeromonas hydrophila,* et l'amorce comprend :
(2) un oligonucléotide ayant la séquence de bases 5' gcggcaggcctaacacatgcaag 3' (SEQ ID N° 2) ; et
(24) un oligonucléotide ayant la séquence de bases 5' atccagccgcaggttcccctac 3' (SEQ ID N° 24).

5. Kit pour la détection de gènes, comprenant :
un support à sondes immobilisées suivant la revendication 1 ; et
un réactif pour détecter une réaction entre les sondes et un acide nucléique cible
dans lequel le réactif contient un ensemble d'amorces comprenant au moins un oligonucléotide choisi parmi les articles (1) à (21) suivants et au moins un oligonucléotide choisi parmi les articles (22) à (28) suivants :
(1) un oligonucléotide ayant la séquence de bases 5' gcggcgtgcctaatacatgcaag 3' (SEQ ID N° 1) ;
(2) un oligonucléotide ayant la séquence de bases 5' gcggcaggcctaacacatgcaag 3' (SEQ ID N° 2) ;
(3) un oligonucléotide ayant la séquence de bases 5' gcggcaggcttaacacatgcaag 3' (SEQ ID N° 3) ;
(4) un oligonucléotide ayant la séquence de bases 5' gcggtaggcctaacacatgcaag 3' (SEQ ID N° 4) ;
(5) un oligonucléotide ayant la séquence de bases 5' gcggcgtgcttaacacatgcaag 3' (SEQ ID N° 5) ;
(6) un oligonucléotide ayant la séquence de bases 5' gcgggatgccttacacatgcaag 3' (SEQ ID N° 6) ;
(7) un oligonucléotide ayant la séquence de bases 5' gcggcatgccttacacatgcaag 3' (SEQ ID N° 7) ;
(8) un oligonucléotide ayant la séquence de bases 5' gcggcatgcttaacacatgcaag 3' (SEQ ID N° 8) ;
(9) un oligonucléotide ayant la séquence de bases 5' gcggcgtgcttaatacatgcaag 3' (SEQ ID N° 9) ;
(10) un oligonucléotide ayant la séquence de bases 5' gcggcaggcctaatacatgcaag 3' (SEQ ID N° 10) ;
(11) un oligonucléotide ayant la séquence de bases 5' gcggatgctttacacatgcaag 3' (SEQ ID N° 11) ;
(12) un oligonucléotide ayant la séquence de bases 5' gcggcgtgcctaacacatgcaag 3' (SEQ ID N° 12) ;
(13) un oligonucléotide ayant la séquence de bases 5' gcggcgtgcataacacatgcaag 3' (SEQ ID N° 13) ;
(14) un oligonucléotide ayant la séquence de bases 5' gcggcatgcctaacacatgcaag 3' (SEQ ID N° 14) ;
(15) un oligonucléotide ayant la séquence de bases 5' gcggcgcgcctaacacatgcaag 3' (SEQ ID N° 15)
(16) un oligonucléotide ayant la séquence de bases 5' gcggcgcgcttaacacatgcaag 3' (SEQ ID N° 16) ;
(17) un oligonucléotide ayant la séquence de bases 5' gcgtcatgcctaacacatgcaag 3' (SEQ ID N° 17) ;
(18) un oligonucléotide ayant la séquence de bases 5' gcgataggcttaacacatgcaag 3' (SEQ ID N° 18) ;
(19) un oligonucléotide ayant la séquence de bases 5' gcgacaggcttaacacatgcaag 3' (SEQ ID N° 19) ;
(20) un oligonucléotide ayant la séquence de bases 5' gctacaggcttaacacatgcaag 3' (SEQ ID N° 20) ;
(21) un oligonucléotide ayant la séquence de bases 5' acagaatgcttaacacatgcaag 3' (SEQ ID N° 21) ;
(22) un oligonucléotide ayant la séquence de bases 5' atccagccgcaccttccgatac 3' (SEQ ID N° 22);
(23) un oligonucléotide ayant la séquence de bases 5' atccaaccgcaggttcccctac 3' (SEQ ID N° 23) ;
(24) un oligonucléotide ayant la séquence de bases 5' atccagccgcaggttcccctac 3' (SEQ ID N° 24) ;
(25) un oligonucléotide ayant la séquence de bases 5' atccagccgcaccttccggtac 3' (SEQ ID N° 25) ;
(26) un oligonucléotide ayant la séquence de bases 5' atccagcgccaggttcccctag 3' (SEQ ID N° 26) ;
(27) un oligonucléotide ayant la séquence de bases 5' atccagccgcaggttctcctac 3' (SEQ ID N° 27) ; et
(28) un oligonucléotide ayant la séquence de bases 5' atccagccgcacgttcccgtac 3' (SEQ ID N° 28).

6. Support à sondes immobilisées pour détecter un gène d'une bactérie pathogène de maladie infectieuse, *Aeromonas hydrophila,* comprenant une pluralité de premières sondes disposées sur un support en phase solide à des intervalles les unes des autres, ladite pluralité de sondes étant choisie parmi les articles (A) à (H) suivants :
(A) une sonde dans laquelle la séquence de bases est représentée par GCCTAATACGTATCAACTGTGACGTTAC (SEQ ID N° 92) ;
(B) une sonde dans laquelle la séquence de bases est représentée par GCCTAATACGTGTCAACTGTGACGTTAC (SEQ ID N° 93) ;
(C) une sonde dans laquelle la séquence de bases est la séquence complémentaire de la séquence de bases représentée par la SEQ ID N° 92 ;
(D) une sonde dans laquelle la séquence de bases est la séquence complémentaire de la séquence de bases représentée par la SEQ ID N° 93 ;
(E) une sonde dans laquelle la séquence de bases est une séquence modifiée d'une longueur de 20 à 30 nucléotides, obtenue par délétion, substitution ou addition de bases sur la séquence de bases représentée par la SEQ ID N° 92 dans la mesure où 80 % ou plus de la séquence de bases sont conservés de manière consécutive et dans la mesure où la séquence modifiée conserve la fonction d'une sonde pour détecter le gène d'*Aeromonas hydrophila* dans les conditions drastiques suivantes : solution d'hybridation : SSPE 6 x/10 % de formamide/cible/0,05 % en poids (SSPE 6 x : NaCl 900 mM, NaH₂PO₄H₂O 50 mM, EDTA 6 mM, pH 7,4) ; conditions d'hybridation : 65°C pendant 3 min, 55°C pendant 4 h, lavage avec SSC 2 x/0,1 % de SDS à 50°C, lavage avec SSC 2 x/0,1 % de SDS à 20°C ;
(F) une sonde dans laquelle la séquence de bases est une séquence modifiée d'une longueur de 20 à 30 nucléotides, obtenue par délétion, substitution ou addition de bases sur la séquence de bases représentée par la SEQ ID N° 93 dans la mesure où 80 % ou plus de la séquence de bases sont conservés de manière consécutive et dans la mesure où la séquence modifiée conserve la fonction d'une sonde pour détecter le gène d'*Aeromonas hydrophila* dans les conditions drastiques suivantes : solution d'hybridation : SSPE 6 x/10 % de formamide/cible/0,05 % en poids (SSPE 6 x : NaCl 900 mM, NaH₂PO₄H₂O 50 mM, EDTA 6 mM, pH 7,4) ; conditions d'hybridation : 65°C pendant 3 min, 55°C pendant 4 h, lavage avec SSC 2 x/0,1 % de SDS à 50°C, lavage avec SSC 2 x/0,1 % de SDS à 20°C ;
(G) une sonde dans laquelle la séquence de bases est une séquence modifiée d'une longueur de 20 à 30 nucléotides, obtenue par délétion, substitution ou addition de bases sur la séquence complémentaire de la séquence de bases représentée par la SEQ ID N° 92 dans la mesure où 80 % ou plus de la séquence complémentaire sont conservés de manière consécutive et dans la mesure où la séquence modifiée conserve la fonction d'une sonde pour détecter le gène d'*Aeromonas hydrophila* dans les conditions drastiques suivantes : solution d'hybridation : SSPE 6 x/10 % de formamide/cible/0,05 % en poids (SSPE 6 x : NaCl 900 mM, NaH₂PO₄H₂O 50 mM, EDTA 6 mM, pH 7,4) ; conditions d'hybridation : 65°C pendant 3 min, 55°C pendant 4 h, lavage avec SSC 2 x/0,1 % de SDS à 50°C, lavage avec SSC 2 x/0,1 % de SDS à 20°C ; et
(H) une sonde dans laquelle la séquence de bases est une séquence modifiée d'une longueur de 20 à 30 nucléotides, obtenue par délétion, substitution ou addition de bases sur la séquence complémentaire de la séquence de bases représentée par la SEQ ID N° 93 dans la mesure où 80 % ou plus de la séquence complémentaire sont conservés de manière consécutive et dans la mesure où la séquence modifiée conserve la fonction d'une sonde pour détecter le gène d'*Aeromonas hydrophila* dans les conditions drastiques suivantes : solution d'hybridation : SSPE 6 x/10 % de formamide/cible/0,05 % en poids (SSPE 6 x : NaCl 900 mM, NaH₂PO₄H₂O 50 mM, EDTA 6 mM, pH 7,4) ; conditions d'hybridation : 65°C pendant 3 min, 55°C pendant 4 h, lavage avec SSC 2 x/0,1 % de SDS à 50°C, lavage avec SSC 2 x/0,1 % de SDS à 20°C ; et
dans lequel le support à sondes immobilisées comprend au moins une seconde sonde ayant l'une quelconque des séquences de bases des SEQ ID N° 35-91, 94 comme mentionné dans la description, immobilisée à une position espacée de la pluralité de premières sondes.

7. Procédé de détection d'un gène d'*Aeromonas hydrophila* dans un analyte en utilisant un support à sondes immobilisées, comprenant les étapes de :
(i) réaction de l'analyte avec un support à sondes immobilisées suivant la revendication 1 ; et
(ii) détection de la présence ou de l'absence d'une réaction de la sonde sur le support à sondes immobilisées avec un acide nucléique dans l'analyte, ou détection de l'intensité d'une réaction d'hybridation de la sonde sur le support à sondes immobilisées avec un acide nucléique dans l'analyte.

8. Procédé suivant la revendication 7, comprenant en outre l'étape de conduite d'une amplification par PCR de l'acide nucléique cible dans l'analyte en utilisant une amorce comprenant les oligonucléotides suivants :
(2) un oligonucléotide ayant la séquence de bases 5' gcggcaggcctaacacatgcaag 3' (SEQ ID N° 2) ; et
(24) un oligonucléotide ayant la séquence de bases 5' atccagccgcaggttcccctac 3' (SEQ ID N° 24).

9. Série de sondes pour la détection spécifique d'un gène d'une bactérie pathogène de maladie infectieuse, *Aeromonas hydrophila,* comprenant deux sondes consistant en les séquences de bases (1) et (2) suivantes, respectivement :
(1) GCCTAATACGTATCAACTGTGACGTTAC (SEQ ID N° 92) ; et
(2) GCCTAATACGTGTCAACTGTGACGTTAC (SEQ ID N° 93).
